# EUROPÄISCHE PATENTSCHRIFT

(11) **EP 1 414 938 B1**
(45) Veröffentlichungstag und Bekanntmachung des Hinweises auf die Patenterteilung: **18.07.2007**
(21) Anmeldenummer: 02787132.6
(22) Anmeldetag: 16.07.2002
(51) Int. Cl.: C12M 1/02, C12M 1/04, C12M 1/12, C12N 1/00, C05F 17/00, C05F 11/08

(54) **VERFAHREN UND VORRICHTUNG ZUR BEREITUNG EINES MIT MIKROORGANISMEN BEFRACHTETEN FLUIDES, MIKROORGANISMEN ENTHALTENDER STOFF SOWIE VERFAHREN ZUR VERÄNDERUNG DER WACHSTUMSBEDINGUNGEN VON PFLANZEN**
METHOD AND DEVICE FOR PREPARING A FLUID LOADED WITH MICROORGANISMS, A SUBSTANCE CONTAINING MICROORGANISMS, AND METHOD FOR ALTERING THE GROWTH CONDITIONS OF PLANTS
PROCEDE ET DISPOSITIF POUR PREPARER UN FLUIDE CHARGE EN MICRO-ORGANISMES, SUBSTANCE CONTENANT DES MICRO-ORGANISMES ET PROCEDE UTILISE POUR MODIFIER LES CONDITIONS DE CROISSANCE DE PLANTES

(30) Priorität: 16.07.2001 DE 10133777; 20.12.2001 DE 10163001
(43) Veröffentlichungstag der Anmeldung: 06.05.2004
(73) Patentinhaber: LAUBOECK, Theodor, D-85662 Hohenbrunn (DE); BÖHME, Dietmar, D-85662 Hohenbrunn (DE); LUNG, Gerhard, D-70619 Stuttgart (DE); BÖHM, Georg, D-85586 Poing (DE); BILLER-AMETSBICHLER, Joseph, D-85580 Poing (DE)
(72) Erfinder: LAUBOECK, Theodor, D-85662 Hohenbrunn (DE); BÖHME, Dietmar, D-85662 Hohenbrunn (DE); LUNG, Gerhard, D-70619 Stuttgart (DE); BÖHM, Georg, D-85586 Poing (DE); BILLER-AMETSBICHLER, Joseph, D-85580 Poing (DE)
(74) Vertreter: Rössig, Rolf
(86) Internationale Anmeldenummer: PCT/EP2002/007921
(87) Internationale Veröffentlichungsnummer: WO 2003/008531

(56) Entgegenhaltungen:
- EP-A- 0 943 677
- US-B1- 6 168 949

## Beschreibung

Die Erfindung betrifft ein Verfahren und eine Vorrichtung zur Bereitung eines mit Mikroorganismen befrachteten Fluides. Die Erfindung betrifft weiterhin einen Mikroorganismen enthaltenden Stoff sowie ein Verfahren zur Veränderung der Wachstumsbedingungen von Pflanzen

In der konventionellen Land- und Gartentechnik ist es üblich, die Wachstumsbedingungen von Pflanzen durch Ausbringung von Düngemitteln und gegebenenfalls auch von Herbiziden, Fungiziden und Pestiziden zu beeinflussen. Zahlreiche, in diesem Zusammenhang ausgebrachte Stoffe, erweisen sich unter ökologischen Gesichtspunkten als bedenklich. Insbesondere bei der großflächigen Anwendung derartiger Stoffe, können nachhaltig nachteilige Auswirkungen für das betroffene Ökosystem, insbesondere eine Beeinträchtigung der Grundwasserqualität auftreten.

Insbesondere alternativ zur Ausbringung wachstumsbeeinflussender chemischer Substanzen im Land- oder Gartenbau ist es möglich, die Wachstumsbedingungen der Pflanzen durch Bodenbearbeitung wie beispielsweise Vertikutieren und/oder durch die Ausbringung natürlicher Düngemittel, insbesondere von Kompost zu verbessern.

Im Unterschied zu industriell hergestellten Düngemitteln, stehen natürliche Düngemittel nur bedingt in ausreichender Menge zur Verfügung. Die Ausbringung derselben bereitet häufig Probleme. Die mit natürlichen Düngemitteln gedüngten Flächen sind im Regelfall unmittelbar nach der Ausbringung des Düngemittels nur eingeschränkt nutzbar.

Unter dem Eindruck dieses Problems wurden in der Vergangenheit auf pflanzlicher Basis hergestellte Düngemittel-Fluide entwickelt, die in flüssiger Form ausgebracht werden können und deren Düngewirkung auf die in diesen Fluiden enthaltenen Kompostextrakte, insbesondere Mikroorganismen zurückgeführt werden kann.

Aus dem US-Patent US 6168949 B1 ist eine Vorrichtung zur Bereitung eines mit aeroben Mikroorganismen durchsetzten Fluides bekannt, wobei diese Vorrichtung einen, in einen Umwälzkreislauf eingebundenen Tank aufweist. Der Tank ist unter Zusatz eines Fermentationsmittels wie beispielsweise Melasse mit Wasser gefüllt. Über den Umwälzkreislauf wird das mit dem Fermentationsmittel versetzte Wasser abgesaugt und über eine Sprühdüsenanordnung oberhalb des Fluidspiegels in den Tank hinein abgesprüht. Mit einem derart bereiteten, mit Mikroorganismen durchsetzten Fluid, kann eine wachstumsfördernde Wirkung von Nutz- oder Kulturpflanzen, insbesondere Gräsern erreicht werden, ohne dass hierbei nachteilige Auswirkungen auf das Ökosystem auftreten.

Der Erfindung liegt die Aufgabe zugrunde, ein Verfahren und eine Vorrichtung zur Bereitung eines mit Mikroorganismen befrachteten Fluides zu schaffen, durch welche bzw. durch welches ein im Hinblick auf eine gewünschte Populationscharakteristik mit Mikroorganismen befrachtetes Fluid auf zuverlässige Weise bereitet werden kann.

Diese Aufgabe wird erfindungsgemäß gelöst durch eine Vorrichtung zur Bereitung eines mit Mikroorganismen befrachteten Fluides, mit einem Behälter zur Aufnahme des mit Mikroorganismen befrachteten Fluides, einer Ableitungseinrichtung zur Ableitung eines Fluidstroms aus dem Behälter und einer Rückleitungseinrichtung zur Rückleitung des über die Ableitungseinrichtung geführten Fluidstromes in den Behälter, wobei der Fluidstrom über eine Anreicherungseinrichtung geführt wird.

Dadurch wird es auf vorteilhafte Weise möglich, die Generierung der Mikroorganismen in dem Fluid nach Maßgabe eines in der Anreicherungseinrichtung aufgenommenen Fermentationsmedium sowie in Abhängigkeit von selektiv variierbaren Fermentationsparametern abzustimmen.

Gemäß der Erfindung ist die Anreicherungseinrichtung außerhalb des Behälters angeordnet. Hierdurch wird es möglich, die Anreicherungseinrichtung auf handhabungstechnisch günstige Weise mit einem Fermentationsmedium zu beschicken. Gegebenenfalls verbrauchtes Fermentationsmedium kann auf einfache Weise ausgetauscht werden. Die Anreicherungseinrichtung wird nachfolgend auch als Extraktor bezeichnet werden.

Alternativ zu der vorangehend beschriebenen Maßnahme oder gegebenenfalls auch in Kombination hiermit, ist es möglich, die Anreicherungseinrichtung - oder eine zusätzliche Anreicherungseinrichtung in dem Behälter - und zwar vorzugsweise derart anzuordnen, dass zumindest ein Teilbereich eines in der Anreicherungseinrichtung aufgenommenen Fermentationsmediums sich oberhalb des Fluidpegels in dem Behälter befindet.

Die Anreicherungseinrichtung umfasst in vorteilhafter Weise eine Aufnahmekammer; wobei in dieser Aufnahmekammer das Fermentationsmedium aufgenommen ist. Das Fermentationsmedium ist gemäß einer besonders bevorzugten Ausführungsform der Erfindung zumindest anteilig aus Kompost gebildet. Das Kompostmaterial kann derart gebunden sein, dass dieses im Rahmen des Fluidkreislaufes nicht in unzulässig hohem Maße ausgeschwemmt wird. Das Kompostmaterial ist vorzugsweise biologisch aktiv und enthält eine Vielzahl lebender aerober Mikroorganismen. Das Kompostmaterial ist vorzugsweise mit diesen Mikroorganismen angereichert.

Zur Zurückhaltung des Kompostmateriales in der Anreicherungseinrichtung ist gemäß einer besonders bevorzugten Ausführungsform der Erfindung eine Rückhalteeinrichtung vorgesehen, die beispielsweise einen Sieb- oder Filterwandungsabschnitt aufweist.

Eine, im Hinblick auf eine besonders hohe Betriebszuverlässigkeit vorteilhafte Ausführungsform der erfindungsgemäßen Vorrichtung ist dadurch gegeben, dass die Anreicherungseinrichtung auf einem, in vertikaler Richtung gesehen unterhalb des Behälters liegenden Höhenniveau angeordnet ist. Hierdurch wird es möglich, eine Zuleitung des der Anreicherungseinrichtung zufließenden Fluidstromes aufgrund des durch die Behälterfüllhöhe bestimmten statischen Drucks zu erreichen. Auf vorteilhafte Weise kann hierbei auf eine, in eine vom Behälter zur Anreicherungseinrichtung führende Rohrleitung eingebundene Pumpe verzichtet werden. Hierdurch ergibt sich eine besonders hohe Robustheit und niedrige Verschmutzungsempfindlichkeit der erfindungsgemäßen Vorrichtung.

Die Rückförderung des durch die Anreicherungseinrichtung hindurchfließenden Fluides in den Behälter, erfolgt jedoch vorzugsweise unter Zwischenschaltung einer Förderpumpe die gegebenenfalls nur phasenweise betrieben wird. Diese Pumpe ist gemäß einer bevorzugten Ausführungsform der Erfindung als Radialpumpe ausgeführt. Die Pumpe kann derart ausgebildet sein, dass die durch einen zum Antrieb der Pumpe vorgesehen Motor generierte Abwärme über die Pumpe in den Fluidkreislauf hinein abgeführt wird. Hierdurch wird es auf verfahrenstechnisch vorteilhafte Weise möglich, das in dem Behälter befindliche Fluid durch die Abwärme der Pumpe zu beheizen. In vorteilhafter Weise ist die Pumpe als perestaltische Pumpe ausgebildet. Hierdurch wird auf vorteilhafte Weise eine Zerstörung der Mikroorganismen durch den Pumpprozess vermieden.

Die Förderpumpe ist vorzugsweise stromabwärts der Anreicherungseinrichtung angeordnet. Hierdurch wird es insbesondere möglich, im Rahmen der Beschickung der Anreicherungseinrichtung etwaiges noch in der Anreicherungseinrichtung verbliebenes Wasser über die Förderpumpe abzusaugen und in den Behälter zurück zuleiten.

Die Anreicherungseinrichtung umfasst gemäß einer besonders bevorzugten Ausführungsform der Erfindung eine Gehäuseeinrichtung die mit einer Deckeleinrichtung versehen ist, wobei die Deckeleinrichtung zur Ein- oder Ausbringung des Fermentationsmediums abnehmbar ist.

Im Inneren der Anreicherungseinrichtung erfolgt die Zuleitung des Fluides vorzugsweise derart, dass dieses unter einem ausreichenden Überdruck in das Fermentationsmedium eintritt und dieses hierbei durchdringt. Es ist möglich, die Zuleitung des Fluides zu dem Fermentationsmedium derart vorzunehmen, dass das Fermentationsmedium im Zutrittsbereich des Fluides im wesentlichen mit dem durch die Füllhöhe des Behälters bestimmten statischen Fluiddruck beaufschlagt wird.

Die Gehäuseeinrichtung zur Aufnahme des Fermentationsmedium kann kasten- oder vorzugsweise kugelförmig ausgebildet sein. Im Falle einer kugelförmigen Ausgestaltung des Gehäuses der Anreicherungseinrichtung ergibt sich eine besonders hohe Druckfestigkeit bei vergleichsweise geringem Bauraumbedarf.

Gemäß der Erfindung ist eine Gasbefrachtungseinrichtung vorgesehen, zur Befrachtung des in dem Behälter aufgenommenen Fluides mit Gas insbesondere Sauerstoff. Hierdurch wird es auf vorteilhafte Weise möglich, die in dem, in dem Behälter aufgenommenen Fluid enthaltenen aeroben Mikroorganismen über einen ausreichend langen Zeitraum am Leben zu erhalten und insbesondere deren Wachstum und Vermehrung zu unterstützen. Die Gasbefrachtungseinrichtung ist gemäß einer besonders bevorzugten Ausführungsform der Erfindung als Sprudeleinrichtung ausgebildet, zur Einleitung des sauerstoffhaltigen Gases in das Fluid in fein verteilter Form. Hierdurch wird es möglich, das zur Sauerstoffbefrachtung vorgesehene Fluid mit dem zugeführten Sauerstoff in innigen Kontakt zu bringen.

Alternativ_zu dieser Maßnahme, oder auch in Kombination hiermit ist es möglich, das rückgeleitete Fluid beispielsweise als Fluidstrahl gerichtet in das im Behälter befindliche Fluid einzustrahlen. Der Fluidstrahl kann durch entsprechende Düsenstrukturen zusätzlich mit Sauerstoff angereichert werden.

Gemäß einer besonders bevorzugten Ausführungsform der Erfindung ist eine Einspeisungseinrichtung vorgesehen, zur Einspeisung einer Nährlösung insbesondere flüssiger Melasse, von Pflanzenextrakten, homöopathischen Präparaten, Additiven, Kelb, natürlichen, tierischen, oder pflanzlichen Extrakten in das im Behälter befindliche Fluid. Hierdurch wird es möglich, das Wachstum und die Vermehrung der Mikroorganismen noch weiter zu unterstützen.

Die Einspeisung der Nährlösung, insbesondere Melasse erfolgt gemäß einer besonders bevorzugten Ausführungsform der Erfindung zeitlich programmgesteuert und zwar vorzugsweise unter Erfassung bestimmter Zustandseigenschaften des in dem Behälter aufgenommenen Fluides.

Zur Einspeisung der Nährlösung bzw. der vorgenannten Stoffe, insbesondere der Melasse/Vinasse in den Fluidkreislauf ist gemäß einer besonders bevorzugten Ausführungsform der Erfindung ein Einspeiser vorgesehen, wobei dieser (Melasse-) Einspeiser ein Aufnahmebehältnis umfasst, und der Innenraum des Aufnahmebehältnisses mit einem Druckmedium beaufschlagbar ist. Hierdurch wird es möglich, auch hoch konzentrierte und damit zähflüssige Nährstoff- oder Melasselösungen auf zuverlässige Weise in den Fluidkreislauf einzuspeisen. Als Druckmedium zur Beaufschlagung der Melasse, kann gemäß einer besonders bevorzugten Ausführungsform der Erfindung Luft verwendet werden, insbesondere jene Luft die auch in fein verteilter Form von unten in den Fluidbehälter eingespeist wird. Alternativ hierzu ist es auch möglich als Druckmedium Wasser zu verwenden. Hierbei wird es möglich, das (Melasse-) Aufnahmebehältnis auf vorteilhafte Weise zu spülen.

Vorzugsweise ist die Melasse in ein flaschen-, kanister- oder kartuschenartiges Transportbehältnis abgefüllt das ohne Umfüllen an den Melasseeinspeiser ankoppelbar ist.

Der zur Beschickung der Anreicherungseinrichtung vorgesehene Kompost ist gemäß einer besonders bevorzugten Ausführungsform der Erfindung als Beschickungseinheit konfektioniert, insbesondere abgepackt. Die Abpackung des Komposts erfolgt vorzugsweise in einem luft- und wasserdurchlässigen Behältnis, insbesondere einem Netz-, Gewebe- oder Filtermaterialsack. Der derart konfektionierte Kompost kann in diesem Sack belassen und in die Anreicherungseinrichtung eingelegt werden. Sobald der Kompost vollständig verbraucht, insbesondere ausgespült ist, kann der in dem Sack oder Beutel verbleibende Kompost mit dem Sack oder Beutel aus der Anreicherungseinrichtung entnommen werden.

Alternativ zu einer derartigen Sack- oder beutelartigen Vorkonfektionierung des Komposts ist es auch möglich, diesen in einem kasten- oder korbartigen Behältnis vorzuhalten. Der derart vorgehaltene Kompost kann dann vorzugsweise in diesem korb- oder kastenartigen Behältnis in die Anreicherungseinrichtung eingesetzt und nach seiner Auswaschung aus der Anreicherungseinrichtung entnommen werden. Der ausgespülte Kompost kann vorzugsweise durch Zusatz ausgewählter Pflanzenreste z.B. Nessel-Granulat reqeneriert werden und erneut zur Zubereitung eines mit Mikroorganismen befrachteten Fluides verwendet werden.

Gemäß einer besonders bevorzugten Ausführungsform der Erfindung ist die Behältereinrichtung isoliert ausgebildet. Hierdurch wird es möglich, eine hinreichende Erwärmung des in der Behältereinrichtung aufgenommenen Fluides durch die Eigenwärmeentwicklung des Fluides sowie gegebenenfalls durch die über die Pumpe zugeführte Wärmeleistung zu erreichen.

Gemäß einer besonders bevorzugten Ausführungsform der Erfindung ist eine zusätzliche Heizeinrichtung vorgesehen, zum Aufheizen des Fluides. Die Heizeinrichtung kann in Form einer Heizspirale in das in dem Behälter befindliche Fluid eintauchen. Eine besonders schonende und unzulässige hohe Spitzentemperaturen vermeidende Erwärmung des Fluides kann dadurch erreicht werden, dass die Heizeinrichtung in die Anreicherungseinrichtung, in die Ableitungseinrichtung oder in die Rückleitungseinrichtung eingebunden ist. Die Heizeinrichtung ist vorzugsweise derart ausgebildet, daß lokal keine unzulässig hohen Temperaturspitzen auftreten. Eine besonders präzise Temperaturführung wird gemäß einem besonderen Aspekt der vorliegenden Erfindung dadurch erreicht, daß die Temperaturverteilung der Heizstruktur durch Halbleiterelemente, insbesondere Dioden geregelt wird.

Vorzugsweise sind in das außerhalb des Behälters geführte Leitungssystem weitere Behandlungseinrichtungen eingebunden, durch welche das in den Behälter zurückströmende Fluid behandelt werden kann. Insbesondere ist es möglich, das Fluid durch ein Magnetfeld hindurch zu führen, wobei durch magnetische Effekte eine Konditionierung des Wassers erfolgen kann. Weiterhin ist es möglich, vorzugsweise durch elektrochemische oder optische Messeinrichtungen bestimmte Eigenschaften des Fluides zu erfassen. Gemäß einer besonders bevorzugten Ausführungsform der Erfindung sind insbesondere Einrichtung zur Erfassung der Temperatur, der Sauerstoffkonzentration, der Leitfähigkeit, der Konzentration insbesondere ausgewählter Mikroorganismen, sowie des Zuckergehaltes des in den Behälter zurückgeleiteten oder in dem Behälter befindlichen Fluides vorgesehen.

Es ist möglich, auf Grundlage der derart gewonnenen Messergebnisse bestimmte Betriebsparmeter der erfindungsgemäßen Vorrichtung zu verändern. So ist es möglich, bei Abweichungen von einer gewünschten Solltemperatur eine Kühlung oder Heizung des Fluides vorzunehmen. Bei Abweichungen der erfassten Sauerstoffkonzentration ist es möglich, die Sauerstoffzufuhr entsprechend zu erhöhen oder zu erniedrigen. Insbesondere auf Grundlage des erfassten Zuckergehaltes ist es möglich, die Einspeisung des Zuckers in das Fluid gesteuert durchzuführen. Insbesondere zur Erfassung des Zuckergehaltes können optische, insbesondere laseroptische oder holographische Effekte herangezogen werden. Gemäß einem besonderen Aspekt der vorliegenden Erfindung erfolgt eine Wichte- oder Öchslegrad-Messung zur Erfassung des spezifischen Gewichtes des Fluides zur Bestimmung der Zusammensetzung des Fluides, insbesondere dessen Zuckergehalt.

Die eingangs angegebene Aufgabe wird erfindungsgemäß auch gelöst durch ein Verfahren zur Bereitung eines mit Mikroorganismen befrachteten Fluides, bei welchem ein Fluidstrom in einer Anreicherungseinrichtung mit einem Fermentierungsmedium in Kontakt gebracht wird und das mit dem Fermentierungsmedium in Kontakt gebrachte Fluid in eine Behältereinrichtung geleitet wird, wobei in der Behältereinrichtung das Fluid mit Sauerstoff befrachtet wird und dem Fluid eine Nährlösung zugeführt wird, zur Ernährung der Mikroorganismen in dem Behälter.

Gemäß einer besonders bevorzugten Ausführungsform der Erfindung wird jener der Anreicherungseinrichtung zugeführte Fluidstrom aus der Behältereinrichtung abgeleitet. Hierdurch wird es insbesondere möglich, den der Anreicherungseinrichtung zugeführten Fluidstrom in der Behältereinrichtung zu erwärmen, so dass eine Beaufschlagung des in der Anreicherungseinrichtung aufgenommenen Fermentierungsmedium mit unzulässig kaltem oder gegebenenfalls auch unzulässig warmem Wasser vermieden werden kann.

Die Temperatur des Fluides in dem Behälter wird gemäß einer besonders bevorzugten Ausführungsform der Erfindung überwacht so dass insbesondere auch in Folge der Aktivität der generierten Mikroorganismen freigesetzte Wärme nicht zu einer Abbremsung des Wachstums der Mikroorganismen oder gar zur Abtötung derselben führen kann. In weiterhin vorteilhafter Weise wird es möglich, anhand der Temperatur des mit Mikroorganismen befrachteten Fluides den Reifegrad des Fluides zu erkennen.

Vorzugsweise wird die Nährlösung nach Maßgabe eines Einspeisungsprogrammes in den Behälter eingespeist. Das Einspeisungsprogramm kann einen bestimmten Zeitverlauf oder auch bestimmte Zustandsparameter der degenerierten mit Mikroorganismen befrachteten Lösung berücksichtigen. Das Einspeisungsprogramm wird vorzugsweise durch eine elektronische, programmierte Rechnereinrichtung abgearbeitet, die mit einer Schnittstelleneinrichtung versehen ist, durch welche ein Datenaustausch mit externen Visualisierungs- und/oder Bedienungseinrichtungen möglich ist.

Die Einspeisung der Nährlösung erfolgt vorzugsweise in Abhängigkeit ausgewählter Eigenschaften des Fluides. Der überwiegende Teil des Fluides besteht vorzugsweise aus Wasser. Dieses Wasser wird vorzugsweise im Rahmen eines vorbereitenden Verfahrensschrittes, insbesondere im Hinblick auf seinen Kalkgehalt, seinen Sauerstoffgehalt und vorzugsweise auch im Hinblick auf seine Temperatur vorbehandelt.

Eine besonders zuverlässige Vorkonditionierung des Fluides wird möglich, indem dieses zunächst in dem in den Behälter eingefüllt und darin konditioniert wird. Alternativ hierzu oder auch in Kombination mit dieser Maßnahme ist es möglich, das Wasser unter Zwischenschaltung der Anreicherungseinrichtung in den Behälter einzufüllen. Hierbei können besonders hohe Extraktionsgrade erreicht werden.

Vorzugsweise wird das in den Behälter zurückgeleitete Fluid derart in den Behälter eingespeist, dass in dem Behälter eine Drallströmung entsteht. Eine derartige Drallströmung kann insbesondere dadurch erreicht werden indem der Behälter derart ausgebildet ist, dass dieser in einer horizontalen Schnittebene einen im wesentlichen kreisförmigen Querschnitt aufweist, wobei die Zuleitung des Fluides in einen randnahen Bereich des Behälters und zwar bezogen auf die Behälterwand tangential gerichtet erfolgt.

Das in der Behältereinrichtung befindliche Fluid wird gemäß einer besonders bevorzugten Ausführungsform des erfindungsgemäßen Verfahrens mit Sauerstoff befrachtet indem der Sauerstoff in fein verteilter Form in das Fluid eingeleitet wird. Diese Einleitung erfolgt vorzugsweise durch Verwendung einer in das Fluid eingetauchten Porösplatte aus welcher unter Überdruck zugeführtes Gas in fein verteilter Form heraustritt.

Alternativ hierzu auch in Kombination mit dieser Maßnahme ist es möglich, das Fluid fein zerstäubt oder auch strahlartig in den Behälter einzusprühen.

Durch das erfindungsgemäße Verfahren wird es möglich, ein mit Mikroorganismen angereichertes Fluid herzustellen, das manuell, durch maschinelle Spritzvorrichtungen oder auch durch Bewässerungsanlagen auf ein zu behandelndes Areal ausgebracht werden kann. Die Erfindung erstreckt sich damit auch auf ein Verfahren zur Veränderung der Wachstumsbedingungen von Pflanzen durch Aufbringung eines Fluides das nach den vorangehend beschriebenen Verfahrensschritten hergestellt ist.

Das in der erfindungsgemäßen Weise bereitete Fluid kann insgesamt zur Aktivierung biomechanischer Prozesse z.B im Land- Garten- und Feldbau sowie in der Forstwirtschaft eingesetzt werden. Gemäß einem besonderen Aspekt der Erfindung wird das Spektrum der in dem Fluid enthaltenen Substanzen mit einem hohen Reinheitsgrad eingehalten. Durch das erfindungsgemäße Verfahren lassen sich damit auch Fluide für medizinische Zwecke, zur Darmflorastimulation oder Reaktivierung bereiten.

Es ist auch möglich, das Spektrum der in dem Fluid enthaltenen Stoffe, Pflanzen, Lebewesen derart abzustimmen, daß durch dieses eine Kompostaktivierung zur Beschleunigung eines Kompostiervorganges erreicht wird. Für das erfindungsgemäße Verfahren ergeben sich damit auch für den Bereich der Abfallwirtschaft Einsatzmöglichkeiten.

Der Erfindung befasst sich weiterhin auch mit der Aufgabe ein Verfahren und eine Vorrichtung der eingangs genannten Art zu schaffen, durch welches bzw. durch welche ein biologisch aktives Fluid besonders hoher Wirksamkeit bereitet werden kann. Diese Aufgabe wird erfindungsgemäß gelöst durch ein Verfahren zur Bereitung eines biologisch aktiven Fluides, bei welchem ein Fluidstrom über eine Reaktoreinrichtung geführt und in der Reaktoreinrichtung mit biologisch aktiven Substanzen befrachtet wird, wobei der befrachtete Fluidstrom in eine Behältereinrichtung geführt wird und das in der Behältereinrichtung befindliche Fluid derart konditioniert wird, dass sich in dem Fluid eine Pilzkultur ausbildet.

Dadurch wird es auf vorteilhafte Weise möglich, ein biologisch aktives Fluid zu schaffen das als galertartige Masse auf die zu düngenden Fläche ausgebracht werden kann.

Gemäß einer besonders bevorzugten Ausführungsform der Erfindung wird das in der Behältereinrichtung aufgenommene Fluid aktiv mit Sauerstoff befrachtet. Diese aktive Befrachtung des Sauerstoffes kann erfolgen, indem z.B. Umgebungsluft in fein verteilter Form in das Fluid eingeleitet wird. Die Einleitung des Sauerstoffes in fein verteilter Form kann insbesondere durch eine Sprudelanordnung erfolgen die im Bodenbereich der Behältereinrichtung angeordnet ist.

Gemäß einer weiteren besonders bevorzugten Ausführungsform der Erfindung erfolgt eine Regelung der Temperatur des Fluides durch Einsatz einer Temperaturregeleinrichtung. Die Temperaturregeleinrichtung umfasst in vorteilhafter Weise eine Heizeinrichtung zum Aufheizen des Fluides, wobei die Heizeinrichtung derart ausgebildet ist, dass die geforderte Wärmemenge in das aufzuheizende Fluid ohne unzulässig hohen Temperaturgradienten in das Fluid eingebracht wird.

Gemäß einer besonders bevorzugten Ausführungsform der Erfindung ist die Behältereinrichtung mit einer Isolierung versehen zur Verminderung des Abflusses der Wärme des in der Behältereinrichtung aufgenommenen Fluides an die Umgebung.

Gemäß einem weiteren Aspekt der Erfindung ist eine Kühleinrichtung vorgesehen, zur selektiven Ableitung von Wärme aus dem Fluid. Hierdurch wird es insbesondere möglich, unzulässig hohe Fluidtemperaturen zu verhindern. Zudem wird es möglich, durch Ableitung definierter Wärmemengen, einen gewünschten Reifetermin des Fluides präzise anzusteueren. Insbesondere wird es möglich, zu verhindern, daß das Fluid unzulässig lange vor dem Ausbringungszeitpunkt bereits den Endreifegrad erreicht hat. Das Kühlsystem kann derart ausgebildet sein, daß dessen Abwärmebereich selektiv mit dem Fluidsystem in Wärmetauschkontakt bringbar ist. So ist es insbesondere möglich, durch eine Kältemittelanlage ein selektiv als heizung oder Kühleinrichtung wirksames System bereitzustellen.

Die Generierung der Pilzkultur wird gemäß einer besonders bevorzugten Ausführungsform der Erfindung dadurch unterstützt, dass für die gewünschte Pilzkultur vorgesehene Pilzstämme im Bereich des Reaktors auf den abgezweigten Fluidstrom aufgebracht werden, wobei im Bereich des Reaktors vorzugsweise den Fluidstrom Nährsubstanzen aufgegeben werden, die das Wachstum der gewünschten Pilzkultur fördern.

In vorteilhafter Weise erfolgt die Auswaschung der Mikroorganismen aus dem Kompostmaterial in dem ausgelagerten Extraktor, wobei der Auswaschprozess derart durchgeführt werden kann, dass auch in dem Extraktor d.h. innerhalb der Kompostmischung eine weitere Vermehrung der Mikroorganismen erfolgen kann. Der Extraktor wird vorzugsweise derart betrieben, dass die darin aufgenommene Kompostmischung zumindest nicht permanent in der zur Auswaschung vorgesehenen Flüssigkeit liegt. In vorteilhafter Weise wird hierzu der Extraktor intervallweise mit dem Auswasch-Wasser beaufschlagt, wobei die Dauer der Beaufschlagungsperioden sowie die zwischen den Beaufschlagungsperioden liegenden Zeitintervalle vorzugsweise unter Berücksichtigung ausgewählter Prozessparameter, insbesondere der Konzentration der Mikroorganismen in dem zur weiteren Vermehrung derselben vorgesehenen Reaktor. Die Beaufschlagung der Kompostmischung in dem Reaktor erfolgt vorzugsweise unter Verwendung wenigstens einer Vollkegeldüse deren Strahlwinkel vorzugsweise im Bereich von 120° liegt. Das der Kompostmischung zugeleitete Spühlmedium wird vorzugsweise im Rahmen eines vorangehenden Prozessschrittes derart intensiv mit Sauerstoff angereichert, dass in der Kompostmischung keine anaeroben Prozesse ablaufen können, insbesondere keine Vermehrung anaerober Bakterien erfolgt. Vorzugsweise handelt es sich bei dem Spülmedium um weitgehend sauerstoffgesättigtes Wasser.

Die Temperatur des in dem zur Vermehrung der Mikroorganismen und zur Speicherung derselben vorgesehenen Reaktor-Behälter liegt vorzugsweise im Bereich von 20-25°. Der Reaktor-Behälter ist vorzugsweise mit einer Isolierummantelung versehen um den Wärmeabfluss aus dem Reaktor-Behälter in die Umgebung zu reduzieren. Gemäß einem besonderen Aspekt der vorliegenden Erfindung wird das in dem Behälter befindliche, mit Mikroorganismen befrachtete Fluid das im Regelfall überwiegend aus Wasser besteht, beheizt. Die Beheizung erfolgt dabei derart, dass der zur Wärmeübertragung an das Fluid im Bereich der Heizeinrichtung herrschende Temperaturgradient nicht größer ist als ein von den Mikroorganismen tolerierter Temperaturgradient. Ein, besonders Mikroorganismenschonende Beheizung des in dem Behälter befindlichen Fluides kann dadurch erreicht werden, dass die zur Sauerstoffversorgung der Mikroorganismen zugeführte Luft beheizt wird. Hierdurch wird zum einen der Sauerstoffeintrag in das Fluid unterstützt und zum andern unzulässig hohe Spitzentemperaturen vermieden. Alternativ hierzu - oder auch in Kombination damit, ist es auch möglich, die Beheizung des, mit Mikroorganismen befrachteten Fluides durch eine Niedertemperaturheizeinrichtung wie sie beispielsweise bei Aquarien Anwendung finden kann, zu erreichen. Eine geeignete Niedertemperaturheizeinrichtung ist vorzugsweise mit einer Ummantelung versehen deren Wärmedurchgangsvermögen unzulässig hohe Oberflächentemperaturen d.h. Oberflächentemperaturen die für Mikroorganismen bedenklich sein könnten, nicht zulässt.

Die Belüftung der in dem Reaktor aufgenommenen, mit Mikroorganismen befrachteten Lösung, erfolgt vorzugsweise im Bodenbereich des Reaktors. Dadurch wird auf vorteilhafte Weise vermieden, dass sich in dem Reaktor Zonen mit unzureichend hoher Sauerstoffkonzentration ausbilden können.

Gemäß einem besonderen Aspekt der Erfindung wird der Reifegrad des mit Mikroorganismen befrachteten Fluides mit einer Messeinrichtung ermittelt, wobei die hierbei ermittelten Messwerte zur Abstimmung der weiteren Prozessführungsparameter Berücksichtigung finden. Gemäß einer besonders bevorzugten Ausführungsform der Erfindung wird der Reifegrad des mit Mikroorganismen befrachteten Fluides durch eine Messung der optischen Dichte desselben bestimmt. Hierdurch wird es insbesondere möglich, die Konzentration einer, in dem Fluid enthalten Nährlösung bzw. den Zuckeranteil zu messen. Die Bestimmung des Reifegrades kann hierbei auf Grundlage einer Betrachtung des Zuckerabbaus des Fluides erfolgen. So ist es möglich, dem zur Auswaschung der Mikroorganismen vorgesehenen Fluid eine vorzugsweise definierte Zuckermenge zuzuführen, so dass sich zu Beginn des Vermehrungsprozesses in dem Fluid eine vorgegebene Zuckerkonzentration einstellt. Diese Anfangszuckerkonzentration kann auf optischen Wege beispielsweise durch Messung der optischen Dichte, gemessen werden. Die Messung kann mit einer Eichprozedur verknüpft sein. Diese Eichprozedur umfasst vorzugsweise die Messung der optischen Dichte des unbefrachteten Fluides, insbesondere Wassers und die Messung der optischen Dichte des befrachteten Fluides, insbesondere Wassers unter Berücksichtigung der Menge jener der optischen Dichte verändernden und durch die Mikroorganismen abzubauenden Substanz. Ergibt beispielsweise die Messung der optischen Dichte des mit Mikroorganismen befrachteten Fluides, dass eine Anfangszuckerkonzentration von beispielsweise 3,7% zwischenzeitlich auf einen Wert von 0,3% abgebaut ist, so kann der Schluss gezogen werden, dass innerhalb des zwischen den beiden Messzeitpunkten liegenden Zeitintervalles die in dem Fluid aufgenommenen Mikroorganismen eine Mächtigkeit erreicht haben, die einen entsprechenden Abbau der Nährlösung ermöglichte. Der zeitliche Verlauf der Abnahme des Nährstoffgehaltes kann dabei als robustes Indiz für die Mächtigkeit der Mikroorganismen d.h. Anzahl der Mikroorganismen in der Lösung dienen.

Das in dem Reaktor befindliche Fluid besteht vorzugsweise überwiegend aus H₂O, Bakterien und Pilzen. Bei den genannten Bakterien handelt es sich vorzugsweise um antagonistische Leitorganismen wie beispielsweise Bazillus SPP und Floris-Pseudomonaden. Die in der Lösung befindlichen Pilze können in allen Stadien, insbesondere auch als Sporen in der Lösung vorliegen. Die Prozessführung erfolgt vorzugsweise derart, dass eine Dominanz der antagonistischen Leitorganismen erreicht wird. Diese Dominanz wird insbesondere durch Einhaltung eines für diese antagonistischen Leitorganismen optimalen Temperaturkorridors sowie beispielsweise auch durch Zusätze, insbesondere Algenpräpararte erreicht. Die Auswaschung der durch das Extraktionsmedium beaufschlagten Kompostmischung erfolgt vorzugsweise solange, bis eine weitgehend Erschöpfung der in der Kompostlösung vorhandenen Menge an Mikroorganismen erreicht wird.

Gemäß einem besonderen Aspekt der vorliegenden Erfindung wird aus dem in dem Reaktor befindlichen, mit Mikroorganismen befrachteten Fluid eine Trockensubstanz extrahiert. Die Extraktion dieser Trockensubstanz erfolgt vorzugsweise durch Trocknungsprozeduren, insbesondere durch Gefrier und/oder Fließbett -Trocknung. Die Parameter des Trocknungsverfahrens sind vorzugsweise auf die Belastbarkeit der Mikroorganismen abgestimmt. Mittels eines derartig generierten Kompostextraktes, wird es möglich, eine mit Mikroorganismen befrachtete Lösung unmittelbar am Einsatzort zeitsparend zu generieren indem das pulverisierte oder auch nur aufkonzentrierte Kompostextrakt in einen Mischbehälter gegeben wird. Als derartiger Mischbehälter kann der vorangehend beschriebene Reaktor-Behälter verwendet werden. Hierdurch wird es möglich, in Verbindung mit der Zugabe von Nährstoffen, insbesondere zuckerhaltigen Substanzen, eine weitere Vermehrung der Mikroorganismen ohne Bedarf nach dem vorangehend beschriebenen Extraktor zu erreichen.

Es ist möglich, den Reaktorbehälter derart aufzubauen, dass dieser zur Generierung des mit Mikroorganismen befrachteten Fluides, selektiv mit oder ohne dem zur Kompostextration vorgesehenen Extraktor betrieben wird. Vorzugsweise ist der Reaktor derart modular aufgebaut, dass der Extraktor selektiv an diesen an oder abgekoppelt werden kann. Bei dieser Modular-Variante ist der Reaktor vorzugsweise derart aufgebaut, dass die in diesem geforderten, zur Vermehrung der Mikroorganismen gewünschten Prozessbedingungen, unabhängig davon eingehalten werden können, ob der Extraktor an den Reaktor angekoppelt ist oder nicht.

Die Einspeisung von Sauerstoff in die in dem Reaktor befindliche Lösung erfolgt vorzugsweise durch Zuleitung von Luft in fein verteilter Form. Gemäß einem besonderen Aspekt der vorliegenden Erfindung erfolgt die Zuleitung der Luft in fein verteilter Form unter Verwendung einer Drehkranz- oder Tellereinrichtung die im Bodenbereich des Reaktors angeordnet ist und eine Düsenanordnung bildet, über welche die Luft unter Drehung der Düsenanordnung um eine vertikale Achse in die mit Mikroorganismen befrachtete Lösung eintreten kann. Der Drehantrieb dieser Drehdüsenanordung erfolgt vorzugsweise unmittelbar, durch die zur Einspeisung in den Reaktor vorgesehene Luft. Hierzu sind die Austrittssdüsen der Drehdüsenanordnung vorzugsweise derart gerichtet, dass diese auf die Düsenanordnung eine in Drehrichtung gerichtete Kraftkomponente generieren.

Gemäß einem weiteren Aspekt der vorliegenden Erfindung wird das Bereitungsverfahren zur Bereitung der mit Mikroorganismen befrachteten Lösung in Abstimmung mit dem Ausbringungstermin gesteuert bzw. geregelt. So ist es beispielsweise möglich, den Zeitpunkt der Einspeisung der Nährlösung oder den zeitlichen Verlauf der Einspeisung, in das in dem Reaktor befindliche Fluid derart festzulegen, dass ein Abbau der eingespeisten Nährlösung bei Erreichung des Ausbringungstermins erfolgt ist bzw. die Maximalkonzentration der Mikroorganismen erreicht wird.

Durch die erfindungsgemäß vorgeschlagene Befrachtung des in dem Reaktor befindlichen Fluides mit Sauerstoff wird es möglich, den Vermehrungsprozess der Mikroorganismen über einen Zeitraum von beispielsweise vierzehn Tagen hin auszudehnen, so dass das mit Mikroorganismen befrachtete Fluid nur in entsprechend gestreckten Zeitintervallen auf die zu behandelnden Behandlungsflächen ausgebracht werden muß.

Die erfindungsgemäße Bereitung des mit Mikroorganismen befrachteten Fluides ermöglicht, insbesondere auf Grundlage der vorgeschlagenen Messung der optischen Dichte des Fluides, eine Kontrolle und Bewertung des momentanen Reifezustands des Fluides. Auf Grundlage der Kontrolldaten ist es möglich, den Auswaschungs- und Vermehrungsprozess durch eine Feedback-Regelung zu unterstützen. Durch diese Verfahrensführung wird es möglich, eine vorgegebene bzw. eine gewünschte Zusammensetzung, insbesondere Konzentration von Mikroorganismen in dem Fluid in einem engen Toleranzbereich zu erreichen. Es ist möglich, die während der Bereitung des Fluides ermittelten Prozessdaten aufzuzeichnen und durch eine Auswertungsprozedur zu visualisieren. Die Einspeisung der Nährlösung kann unter Heranziehung von Kennfeldern optimiert werden, so dass während des Vermehrungsprozesses in dem Reaktor stets die für das momentane Verfahren günstigsten Vermehrungsbedingungen existieren.

Das erfindungsgemäß bereitete, mit Mikroorganismen befrachtete Fluid, kann im Rahmen einer Direktanwendung im flüssigen Zustand auf Bedarfsflächen aufgebracht werden. In gegebenenfalls verdünnter Form eignet es sich auch zur Blattbehandlung, insbesondere als Präventivmaßnahme. In gegebenenfalls verdünnter Form kann es auch zur Saatgutbehandlung zur Tauchbadbehandlung von ruhnende Pflanzenteilen, insbesondere Knollen und Zwiebeln, verwendet werden.

Das erfindungsgemäße Fluid kann in flüssiger oder auch in konzentrierter, insbesondere pulverisierter Form auch zur Bodenapplikation herangezogen werden. durch eine mit dem erfindungsgemäß bereiteten Fluid oder eines hieraus generierten Konzentrats wird eine Zuimpfung der Behandlungfläche bzw. des Subtrats möglich. Die Zuimpfung kann durch Behandlung des Saatgut oder auch durch Direktbehandlung des Substrats erfolgen. Zur Behandlung von Saatgut ist es möglich, ein aus dem erfindungsgemäß bereiteten Fluid gewonnenes Trocken-Extrakt z.B. durch eine Vakuumsapplikation in die Aussenhülle von Saatgut einzubringen.

Das aus dem erfindungsgemäß bereiteten Fluid gegebenenfalls gewonnene Trocken-Extrakt ist ein lagerfähiges, Kompost-Teepulver aus welchem bedarfsweise auch in Kleinmengen entsprechender Kompost-Tee bereitet werden. Die Ausbringung dieses Kompost-Tees kann durch Spritzgeräte, im Gießverfahren oder durch Bewässerungsanlagen erfolgen.

Ein besonderer Vorteil der erfindungsgemäß vorgeschlagenen Bereitung eines mit Mikroorganismen befrachteten Fluides liegt darin, dass dieses Fluid aus Eigenmaterialien hergestellt werden kann. Das erfindungsgemäß bereitete Fluid eignet sich insbesondere zur Behandlung von Erdsubstraten mit artifiziellem Aufbau insbesondere sandhaltigen Böden. Durch das erfindungsgemäße Fluid werden derartige Böden biologisch aktiviert, wodurch sich für die darauf gedeihenden Pflanzen verbesserte Wachstumsbedingungen ergeben. In Verbindung mit einem zur Auswaschung vorgesehenen Kompostmaterial definierter Qualität wird auch die Qualität des damit bereiteten, mit den extrahierten Mikroorganismen befrachteten Fluides gewährleistbar. Durch die erfindungsgemäße Verfahrensführung wird die thermische und auch die mechanische Belastung der extrahierten Mikroorganismen in vorteilhafter Weise so gering gehalten. Zur Kreislaufdurchführung vorgesehene Pumpen, Düsen und Heizeinrichtung sind vorzugsweise derart ausgebildet, dass durch diese keine bzw. allenfalls eine vernachlässigbare Beeinträchtigung der Mikroorganismen erfolgt. Ein besonders hoher Extraktionsgrad kann erreicht werden, indem der Reaktor stufenweise befüllt wird d.h. ein erster Füllzustand von beispielsweise 60% wird im Rahmen eines ersten Befüllabschnittes erreicht bei welchem in vorteilhafter Weise das zur Befüllung vorgesehene Wasser bereits über das Extraktionsmedium in den Reaktor geleitet wird. Nach Befüllung des Reaktors bis zu dem angegebenen Füllstand können mehrere Extraktionszyklen erfolgen bei welchem das in dem Behälter befindliche und bereits mit Mikroorganismen befrachtete Fluid über den Extraktor umgewälzt wird. Nach einer vorgegebenen Spüldauer von beispielsweise 4 Tagen kann eine weitere Erhöhung des Füllstandes des Reaktors erreicht werden, wobei hierzu das dem Reaktor zugeführte Wasser wieder über den Extraktor geführt wird. Im Rahmen des weiteren Vermehrungsprozesses der Mikroorganismen kann auf eine Umwälzung über den Extraktor verzichtet werden und die Vermehrung der Mikroorganismen in dem Behälter ausschließlich durch Zufuhr von Sauerstoff und Nährlösungen unterstützt werden.

Durch die erfindungsgemäß extrahierten Mikroorganismen wird es alternativ oder in Verbindung mit der Zufuhr von organischen Substanzen zu Nutz- oder Behandlungsflächen, möglich, durch selektiv propagierte antagonistische Mikroorganismen die Wachstumsbedingungen für ausgewählte Pflanzengattungen gezielt zu unterstützen. Antagonistische Mikroorganismen können dabei als Gegenspieler zu pflanzenparasitären Pilzkrankheiten aktiv werden. Der Wirkmechanismus der antagonistischen Mikroorganismen basiert auf der Konkurrenz um Nährstoffe um Lebensraum mit den pflanzenparasitären Pilzen sowie der Antibiosis/Lysis durch antibiotische Metaboliten, Enzyme, lytische Agenzien, spezifische und unspezifische Metaboliten von fungitiven Substanzen. Der Wirkmechanismus von antagonistischen Bakterien in Bezug auf die Pflanze schließt die Förderung des Pflanzenwachstum über Pytohormone, über diverse Metaboliten sowie durch Förderung der Toleranzen gegen Krankheiten und Stress ein.

Das zur Befüllung des Extraktors vorgesehene Kompostmaterial kann in vorteilhafter Weise durch den Anwender selbst hergestellt werden. Verwendet werden kann z.B. Kompost der einem ausreichenden Kompostierungsprozess unterzogen wurde. Hierbei ist es von Vorteil wenn der Kompost aus Material bekannter Herkunft bereitet wird. Bei der Verwendung von gegebenenfalls infiziertem Pflanzenmaterial ist darauf zu achten, dass das zu kompostierende Material vorübergehend hinreichend hohe Kompostierungstemperaturen in der Kompostmiete erfahren hat, damit im Extraktor keine Vermehrung von etwaigen Patogenen erfolgt. Das verwendete Kompostmaterial sollte frei von Kompostwürmern sein damit diese beim Extrahierungsprozess die Leitungen bzw. Pumpen und Filter nicht verstopfen. Als zu kompostierendes Material hat sich insbesondere Grüngutmaterial das auch mit Stallmistgaben angereichert sein kann, erwiesen. Besonders hohe Extraktionsergebnisse können auch mit Rindenkompostmaterial erreicht werden das sich zudem durch eine hohe Regenerationsfähigkeit auszeichnet.

Es ist auch möglich, das zur Extraktion der Mikroorganismen vorgesehene Kompostmaterial als im Rahmen einer Qualitätskontrolle geprüftes Kompostmaterial zu beziehen. Hierdurch wird es vor allem möglich, eine Freiheit von Patogenen, einen gewünschten Zustand des Kompost, insbesondere dessen Rottegrad sowie die Zusammensetzung des Ausgangsmateriales zu gewährleisten.

Als Zutaten für die Vermehrung der extrahierten Mikroorganismen eignen sich insbesondere Melasse/Vinassesubstanzen. Bei der vorzugsweise verwendeten Melasse/Vinasse handelt es sich vorzugsweise um, bei der Zuckergewinnung anfallende Melasse/Vinasseformen. Es können auch Melasse/Vinasseformen eingesetzt werden, die speziell für die Zubereitung der mit komposttypischen Mikroorganismen befrachteten Lösung mit Zusätzen aufbereitet sind.

Als weitere Zusätze können auch Algenpräparate Anwendung finden. Insbesondere können Algen in flüssiger oder pulverförmiger Form zur Anwendung kommen. Pulverförmige Algenprodukte werden vorzugsweise vor Zugabe in den Reaktor aufgeschwemmt und gegebenenfalls abgefiltert um Verstopfungen von Leitungen, Pumpen und Düsen zu vermeiden. Es ist auch möglich, dem in dem Reaktor befindlichen Fluid Mikronährstoffe zuzuführen.

Zur Bereitung einer Menge von 1000 Litern eines mit Mikroorganismen befrachteten Fluides werden vorzugsweise 10 kg Kompostmaterial, 4 l Melasse/Vinasse, 1,0 l Algenextrakt und gegebenenfalls 0,5 I Mikronährstoffe verwendet. Der zur Aufnahme der mit Mikroorganismen befrachteten Flüssigkeit vorgesehene Reaktor wird vorzugsweise mit 1000 Wasser befüllt die unter Zwischenschaltung des Extraktors in den Reaktor eingeleitet werden. Die Zugabe der Melasse/Vinasse und des Algenextraktes sowie gegebenenfalls der Mikronährstoffe kann in dem Reaktor oder auch innerhalb des zur Umwälzung vorgesehenen Leitungssystemes erfolgen.

Bei der mit Mikroorganismen befrachteten Flüssigkeit handelt es sich in vorteilhafter Weise um einen Kompost-Tee der als Pflanzenstärkungsmittel zum Einsatz kommen kann. Der Kompost-Tee enthält nützliche Mikroorganismen aus der Gruppe der Antagonisten die gegen parasitäre Pilzkrankheiten wirksam sind. Der erfindungsgemäß bereitete Kompost-Tee eignet sich insbesondere zur Behandlung von Rasentragschichten sowie zur Wachstumsförderung von Nutzpflanzen im Bereich der Landwirtschaft insbesondere im Bereich des Gemüsebaus.

Weitere Einzelheiten der Erfindung ergeben sich aus der nachfolgenden Beschreibung in Verbindung mit der Zeichnung.

Es zeigen:
- **Fig. 1a**: eine vereinfachte Darstellung eines erfindungsgemäßen Systems mit einer außerhalb einer Behältereinrichtung angeordneten Anreicherungseinrichtung (Extraktor);
- **Fig 1b**: eine vereinfachte Darstellung eines erfindungsgemäßen Systems ebenfalls mit einer außerhalb der Behältereinrichtung angeordneten Anreicherungseinrichtung (Extraktor) mit abgewandeltem Aufbau;
- **Fig. 1c**: eine Schemadarstellung einer dritten Variante eines erfindungsgemäßen Systems mit einer im Bodenbereich der Behältereinrichtung (Reaktor) angeordneten Lufteinleitungseinrichtung zur Zuleitung beheizter Luft;
- **Fig. 1d**: eine Schemadarstellung einer vierten Variante eines erfindungsgemäßen Systems mit einer im Bodenbereich der Behältereinrichtung (Reaktor) angeordneten Lufteinleitungseinrichtung und einer Heiz/Kühleinrichtung zum selektiven Aufheizen/Kühlen des Fluides;
- **Fig. 2**: eine perspektivische Ansicht einer in kastenbauweise aufgebauten Anreicherungseinrichtung wie sie beispielsweise bei dem in Fig. 1 dargestellten System Anwendung finden kann;
- **Fig. 3**: eine Datailansicht des Bodenbereich der Behältereinrichtung mit integrierter Gaseinleitungsvorrichtung;
- **Fig. 4**: eine perspektivische Ansicht einer erfindungsgemäßen Tauchlanzenanordnung zur Sauerstoffanreicherung und zur Fermentierung insbesondere Mikrobenbefrachtung von Wasser;
- **Fig. 5**: ein weiteres Ausführungsbeisjpiel mit einem Topfgehäuse zur Aufnahme eines aus einem wasserdurchlässigen Sackmaterial gefertigten und mit Kompost gefüllten Kompostsackes;
- **Fig. 6**: eine weitere Ausführungsform einer Anreicherungseinrichtung mit einem kugelförmigen Druckgehäuse und einem darin angeordneten Aufnahmekorb zur Aufnahme einer Kompostfüllung.

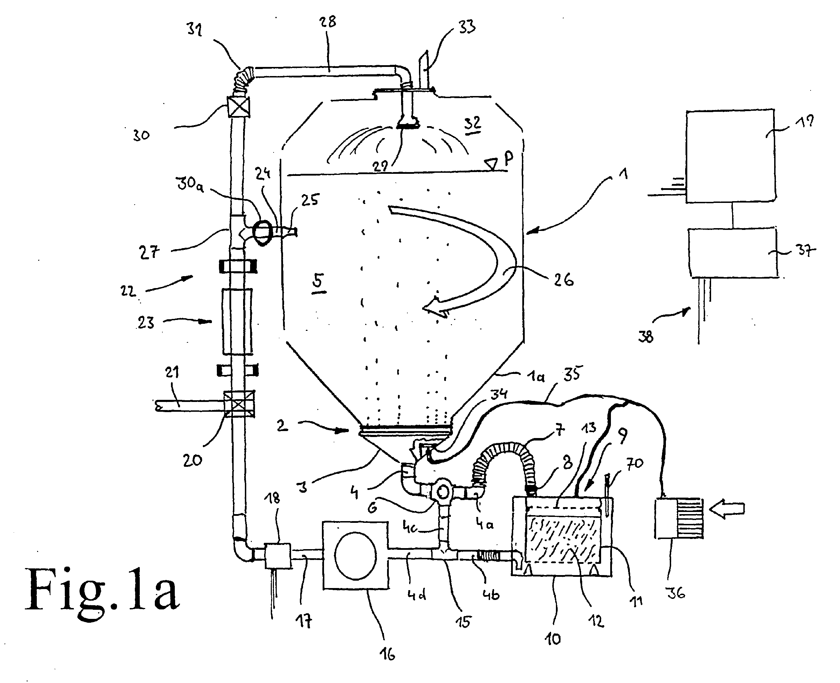

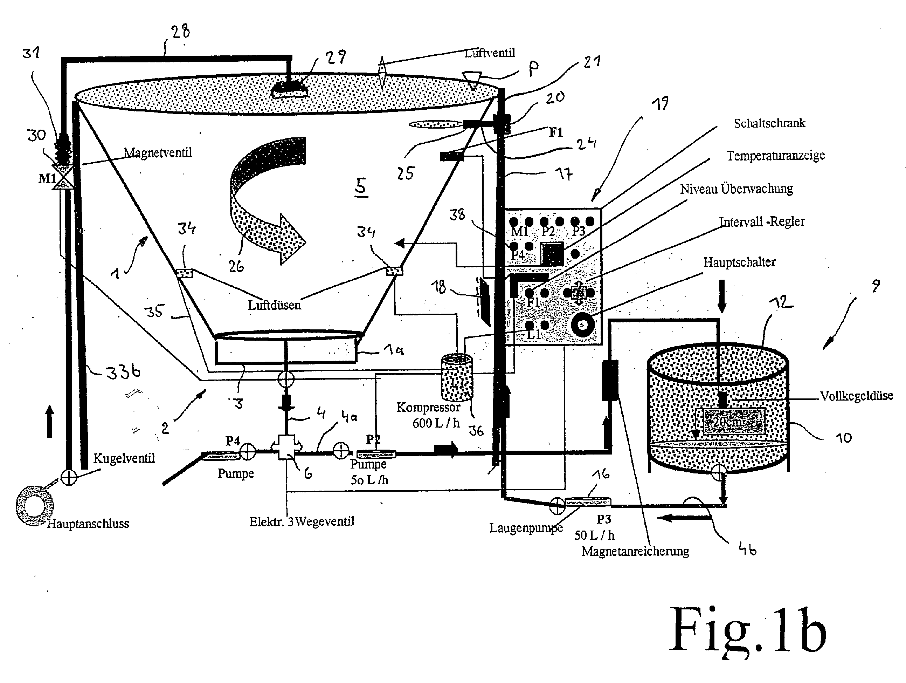

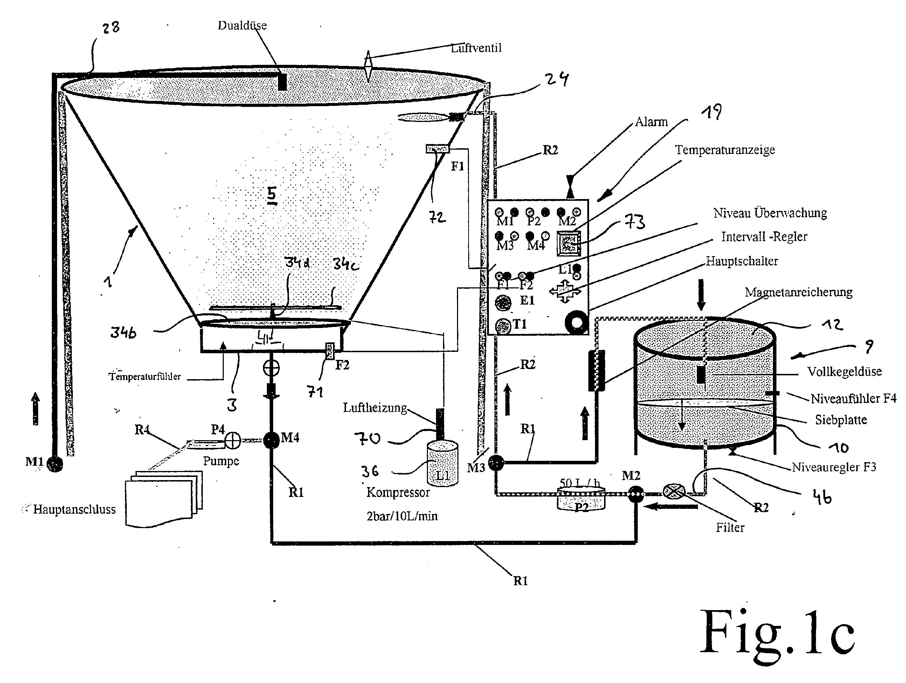

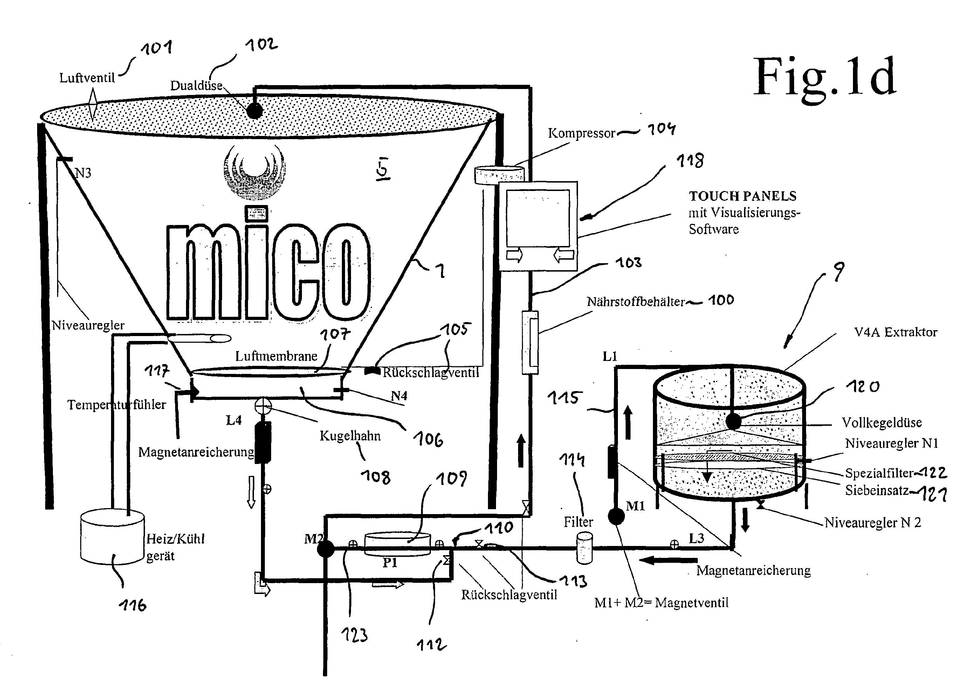

Das in Fig. 1 a dargestellte System umfasst eine Behältereinrichtung 1 die in einem hier nicht näher dargestellten standfesten Gestell aufgenommen ist. Die Behältereinrichtung 1 weist einen sich trichterartig zu einer unteren Bodengruppe 2 hin verjüngenden Bodenbereich 1a auf. Die Bodengruppe 2 umfasst eine Bodenverschlussanordnung 3 die lösbar an der Behältereinrichtung 1 befestigt ist. Im Bereich der Bodenverschlussanordnung 3 ist eine Ablaufleitung 4 angeschlossen über welche ein in der Behältereinrichung 1 aufgenommenes Fluid 5 aus der Behältereinrichtung 1 abgeleitet werden kann.

Bei dem hier dargestellten Ausführungsbeispiel führt die Ablaufleitung 4 zu einem Mischventil 6, über welches eine Aufteilung des über die Ablaufleitung 4 abgezweigten Fluidstromes möglich ist. Von dem Mischventil 6 führt ein weiterer Abschnitt der Ablaufleitung 4a über ein flexibles Verbindungsstück 7 und über eine Schnellverschlusskupplung 8 zu einer Anreicherungseinrichtung 9. Die Anreicherungseinrichtung 9 umfasst ein Aufnahmegehäuse 10 das bei der hier dargestellten Ausführungsform kastenartig ausgebildet ist und sich in vertikaler Richtung gesehen deutlich unterhalb des oberen Flüssigkeitspegels P des in der Behältereinrichtung 1 aufgenommenen Fluides 5 befindet. In dem Aufnahmegehäuse 10 ist ein wasserdurchlässiges Kompostbehältnis 11 angeordnet in welchem eine Kompostfüllung 12 aufgenommen ist. Die Kompostfüllung 12 wird bei diesem Ausführungsbeispiel von oben her durch das über den Ablaufleitungsabschnitt 4a zugeleitete Fluid beaufschlagt.

Zur Vergleichmäßigung der Fluidbeaufschlagung des der Kompostfüllung 12 ist oberhalb derselben eine Siebplatte 13 angeordnet. Das aus der Kompostfüllung 12 ablaufende, durch Bestandteile der Kompostfüllung sowie mit Mikroorganismen befrachtete Fluid wird über einen Saugleitungsabschnitt 4b aus der Anreicherungseinrichtung abgesaugt.

Der Absaugleitungsabschnitt 4b wiederum ist mit einem vom Mischventil 6 ausgehenden Beipassleitungsabschnitt 4c über ein Verbindungsstück 15 zu einem gemeinsamen Saugleitungsabschnitt 4d zusammengeführt. Der Saugleitungsabschnitt 4d führt zu einer Umwälzpumpe 16, wobei die Umwälzpumpe 16 mit einem Isolationsgehäuse versehen ist, so dass die durch einen Antriebsmotor der Umwälzpumpe 16 generierte Abwärme in das durch die Umwälzpumpe 16 geförderte Fluid hinein abgegeben wird. An den Ausgang der Umwälzpumpe 16 ist eine Rückleitungseinrichtung 17 angeschlossen, über welche das durch die Umwälzpumpe 16 geförderte Fluid bedarfsweise in den Behälter 1 zurück geführt werden kann. In die Rückleitungseinrichtung 17 ist bei dem dargestellten Ausführungsbeispiel eine Messanordnung 18 eingekoppelt über welche ausgewählte Eigenschaften des Fluides erfasst werden können.

Die Messanordnung 18 umfasst bei dem hier gezeigten Ausführungsbeispiel einen Sauerstoffsensor zur Erfassung des Sauerstoffgehaltes des zurückgeleiteten Fluides sowie einen Messkopf zur Erfassung des Zuckergehaltes des Fluides. Die durch die Messanordnung 18 generierten Messsignale werden von einer zentralen Steuereinrichtung 19 erfasst und zur Prozesssteuerung verwendet. In der Rückleitungseinrichtung 17 ist weiterhin ein Abzweigventil 20 vorgesehen über welches selektiv das durch die Umwälzpumpe 16 geförderte Fluid in eine Entnahmeleitung 21 geleitet werden kann. Die Rückleitungseinrichtung 17 führt bei dem gezeigten Ausführungsbeisjpiel durch weitere Fluidbehandlungseinrichtungen 22,23 durch welche das Fluid beheizt oder wie hier angedeutet magnetisch behandelt werden kann. Die Rückleitungseinrichtung 17 steht mit einer Kesselspeiseleitung 24 in Verbindung, über welche das Fluid in die Behältereinrichtung 1 eingeleitet werden kann. Die Kesselspeiseleitung 24 mündet über eine Kesselspeisedüse 25 in den Innenraum der Behältereinrichtung 1. Die Kesselspeisedüse 25 ist derart ausgerichtet und angeordnet, dass diese das zurückgeleitete Fluid derart tangential in die Behältereinrichtung 1 einstrahlt, dass das in der Behältereinrichtung 1 befindliche Fluid in eine Drallströmung versetzt wird. Bei dem hier gezeigten Ausführungsbeisjpiel ist die Düse derart angeordnet dass sich ein Rechtsdrall ergibt, wie dies durch das Pfeilsymbol 26 angedeutet ist. Alternativ zu der Einspeisung des zurückgeleiteten Fluides in die Behältereinrichtung 1 über die tangential ausgerichtete Kesselspeisedüse 25 oder auch - wie bei diesem Ausführungsbeispiel gezeigt- in Kombination hiermit ist es möglich, die Rückleitungseinrichtung 17 über eine Verzweigungsstelle 27 an einen weiteren Rücklaufleitungsabschnitt 28 anzuschließen über welchen das geförderte Fluid beispielsweise über einen Sprühkopf 29 in fein verteilter Form auf die Fluidoberfläche des in der Behältereinrichtung 1 bevorrateten Fluides abgesprüht werden kann. Bei dem hier dargestellten Ausführungsbeispiel ist vor dem Rücklaufleitungsabschnitt 28 ein Absperrventil 30 sowie ein flexibles Verbindungsstück 31 vorgesehen. Der oberhalb des Flüssigkeitsspiegels P in der Behältereinrichtung 1 befindliche Luftraum 32 wird über eine Druckausgleichsleitung 33b bedarfsweise entlüftet. In dem Luftraum 32 herrscht bei dem hier dargestellten Ausführungsbeispiel im wesentlichen atmosphärischer Druck. Gegebenenfalls über die Entlüftungsleitung 33 abströmende Gase können einer Geruchsfiltereinrichtung beispielsweise einer Aktiv-Kohlefiltereinrichtung zugeführt werden.

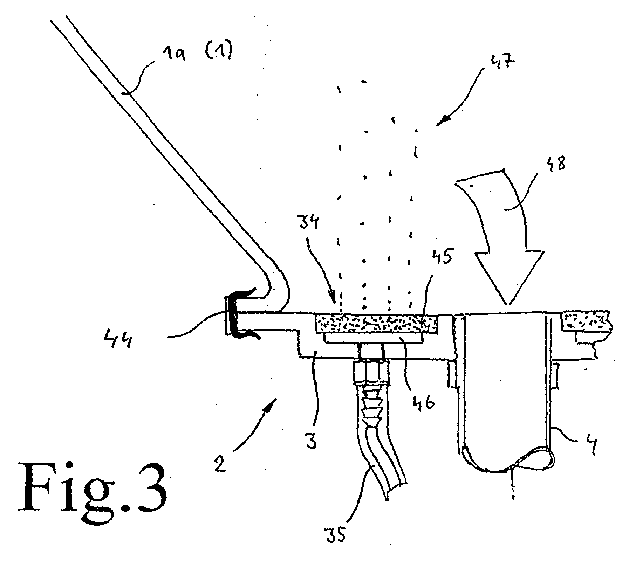

Im Bereich der Bodenverschlussanordnung 3 ist eine Gaseinleitungsvorrichtung 34 vorgesehen, durch welche Druckluft oder gegebenenfalls auch Rein-Sauerstoff in fein verteilter Form in die, in der Behältereinrichtung 1 befindliche Flüssigkeit eingespeist werden kann. Die Gaseinleitungsvorrichtung umfasst bei dem hier gezeigten Ausführungsbeispiel eine, später noch in Verbindung mit Fig. 3 beschriebene, aus einem Sintermaterial gefertigte Porösplatte und einen Druckluftleitungsanschluss an welchen eine Druckluftleitung 35 angeschlossen ist. Die Druckluftleitung 35 ist an eine Kompressoreinrichtung 36 angeschlossen, über welche gefilterte Umgebungsluft, vorzugsweise unter Zwischenschaltung eines Sauerstoffseperators angesaugt und gefördert werden kann. Der Betrieb der Kompressoreinrichtung 36 wird vorzugsweise ebenfalls durch die Steuereinrichtung 19 gesteuert. Über die Steuereinrichtung 19 werden bei dargestellten Ausführungsbeispiel insbesondere das Mischventil 6, die Umwälzpumpe 16 sowie das Absperrventil 30 und das Absperrventil 30a unter Berücksichtigung der seitens der Messanordnung 18 generierten Messsignale gesteuert. Die zeitliche Entwicklung bestimmter Zustandsgrößen des in der Behältereinrichtung 1 im Zusammenspiel mit der Anreicherungseinrichtung 9 generierten Fluides wird durch eine in die Steuereinrichtung 19 integrierte Speichereinrichtung aufgezeichnet und nach Massgabe eines ebenfalls auf der Steuereinrichtung 19 abgespeicherten Programmes unter Verwendung einer Anzeigeeinrichtung 37 visualisiert. die seitens der Steuereinrichtung aufgezeichneten Informationen können über eine Schnittstelleinrichtung 38 abgefragt werden. über diese Schnittstelleneinrichtung 38 ist es auch möglich, auf den Steuerungsablauf Einfluss zu nehmen. Die Schnittstelleneinrichtung 38 ist vorzugsweise als USB oder RS 223-Schnittstelle ausgebildet. Alternativ hierzu oder in besonders vorteilhafter Weise auch in Kombination hiermit sind weiterhin Schnittstelleneinrichtungen vorgesehen über welchen ein Signaltransfer beispielsweise über ein Telefonnetz oder eine Internetdatenleitung möglich ist. Hierdurch wird es möglich die Generierung des in der Behältereinrichtung 1 gebildeten, mit Mikroorganismen befrachteten Fluides im Rahmen einer Fernüberwachung zu steuern und bedarfsweise zu verfolgen.

Die Anreicherungseinrichtung 9 und vorzugsweise auch die Umwälzpumpeneinrichtung 16 sind gemäß einer besonders bevorzugten Ausführungsform der Erfindung zu einer selektiv an die Behältereinrichtung 1 andockbaren modularen Einheit zusammengefasst so dass unter Verwendung dieser modularen Einheit abfolgend in mehreren Behältereinrichtungen mit Mikroorganismen befrachtete Fluidmischungen bereitgestellt werden können. Die Sauerstoffversorgung kann in diese Einheit integriert - oder vorzugsweise für jeden Behälter individuell vorgesehen sein. Im letzteren Fall wird es möglich, auch nach Abkoppelung des Moduls eine ausreichende Sauerstoffversorgung zu gewährleisten.

Fig. 1b zeigt eine, im insbesondere im Hinblick auf die Leitungsführung gegenüber der vorangehend beschriebenen Ausführungsform veränderte Variante. Für die in dieser Darstellung dargestellten Komponenten, insbesondere die mit Bezugszeichen versehenen Komponenten, gelten die Ausführungen zu Fig. 1a sinngemäß.

In Fig. 1c ist eine, um eine Luftheizungseinrichtung 70 erweiterte Ausführungsform des erfindungsgemäßen Systems dargestellt, wobei auch hier, insbesondere für die durch Bezugszeichen gekennzeichneten Komponenten die vorangehenden Ausführungen sinngemäß gelten. Im Bodenbereich der Behältereinrichtung 1 ist eine Düsenanordnung 34b vorgesehen durch welche die über die Kompressoreinrichtung 36 geförderte und die durch die Heizungseinrichtung 70 beheizte Luft in fein verteilter Form in das in der Behältereinrichtung 1 aufgenommene Fluid eingeleitet werden kann.

Die Düsenanordnung kann auch an einem in dem Fluid 5 drehbar angeordneten Drehdüsenelement 34c beispielsweise einem Drehteller- oder Drehkreuzelement ausgebildet sein. das Drehdüsenelement 34c ist bei diesem Ausführungsbeispiel über eine Lageranordnung 34d derart drehbewegbar gelagert, dass dieses um eine im wesentlichen vertikal verlaufende Achse drehbar ist. An dem Drehdüsenelement 34c sind mehrere Abstrahlöffnungen derart ausgebildet, dass das Drehdüsenelement 34c infolge der Abstrahlung der Luft in das Fluid 5 tangentiale Kraftkomponenten erfährt und hierdurch in Drehung versetzt wird. Die Lagereinrichtung 34d umfasst ein Luftkissenlager durch welches das Drehdüsenelement 34c leichtgängig drehbewegbar gelagert ist. Die Temperatur des in der Behältereinrichtung 1 aufgenommenen Fluides wird bei diesem Ausführungsbeispiel durch eine im Bodenbereich der Behältereinrichtung 1 angeordnete Temperaturerfassungseinrichtung 71 und eine zweite im oberen Bereich der Behältereinrichtung 1 angeordnete zweite Temperaturerfassungseinrichtung 72 erfasst. die durch die beiden Temperaturerfassungseinrichtungen 71, 72 generierten Signale werden seitens der Steuereinrichtung 19 bei der Ablaufsteuerung berücksichtigt und über die hier angedeutet dargestellte Temperaturanzeige 73 visualisiert.

In Fig. 1d ist eine, um ein Heiz/Kühlgerät und einen Nährstoffbehälter 100 erweiterte Ausführungsform des erfindungsgemäßen Systems dargestellt, wobei auch hier, die vorangehenden Ausführungen sinngemäß gelten.

Das System umfaßt ein Luftventil 101 , und eine Dual- oder Dralldüse 102 die über einen Zuleitungsabschnitt 103 in welchem der Nährstoffbehälter 100 angeordnet ist, angeströmt wird.

Aus einem oberen Bereich des Behälters 1 wird Luft über einen Kompressor 104 abgezogen und über ein Rückschlagventil 105 in einen Einspeiseabschnitt 106 geführt. Der Einspeiseabschnitt befindet sich im Bodenbereich der Behältereinrichtung 1 und ist vom Fluidraum durch eine luftdurchlässige Membrane 107 getrennt.

Über eine Absperreinrichtung 108 ist es möglich einen Fluidstrom aus der Behältereinrichtung 1 abzuleiten. Der abgeleitete Fluidstrom gelangt vorzugsweise durch ein Magnetfeld hindurch zu einer Pumpe 109 und kann über diese in den Leitungsabschnitt 103 eingespeist werden. Über eine Verzweigungsstelle 110 ist es möglich über die Pumpe 109 auch das aus der Anreicherungseinrichtung 9 abfließende Fluid zu fördern. Die stromaufwärts der Verzweigungsstelle angeordneten Leitungsabschnitte sind jeweils mit einer Rückschlagventileinrichtung 112 113 versehen. Zwischen der Pumpe 109 und der Anreicherungseinrichtung 9 befindet sich eine Filtereinrichtung 114. Die Beschickung der Anreicherungseinrichtung 9 mit Fluid erfolgt über einen Leitungsabschnitt 115 der über eine Magnetventileinrichtung M1 selektiv an einen stromabwärts der Pumpe 109 liegenden Leitungsabschnitt 123 oder an den Behälter 1 oder einen mit diesem kommunizierenden Leitungsabschnitt ankoppelbar ist.

Die Temperatur des Fluides 5 kann über ein Heiz/Kühlgerät 116 einem vorgegebenen zeitlichen Temperaturverlauf angepasst werden. Das Heiz/Kühlgerät ist derart ausgebildet, daß dessen Heiz- oder Kühlabschnitte selektiv mit dem Fluid in Wärmetauschkontakt bringbar sind.

Die Temperatur wird vorzugsweise durch Temperaturfühler 117 erfasst und einer Steuereinrichtung 118 mitgeteilt. Die Steuereinrichtung 118 ist mit einer Displayeinrichtung versehen, zur Ausgabe Prozessindikativer Informationen.

In der Anreicherungseinrichtung 9 ist eine Düseneinrichtung 120 vorgesehen, zum Abstrahlen eines Vollkegel-Nebels auf ein Extraktionsmaterial (nicht dargestellt) Das Extraktionsmaterial sitzt auf einem Siebeinsatz 121 unter Zwischenlage eines Filters 122 auf. Der Pegelstand in der Anreicherungseinrichtung 9 wird über einen Niveauregler N2 eingestellt.

De Füllstand in der Behältereinrichtung 1 wird durch einen Niveauregler N3 eingestellt.

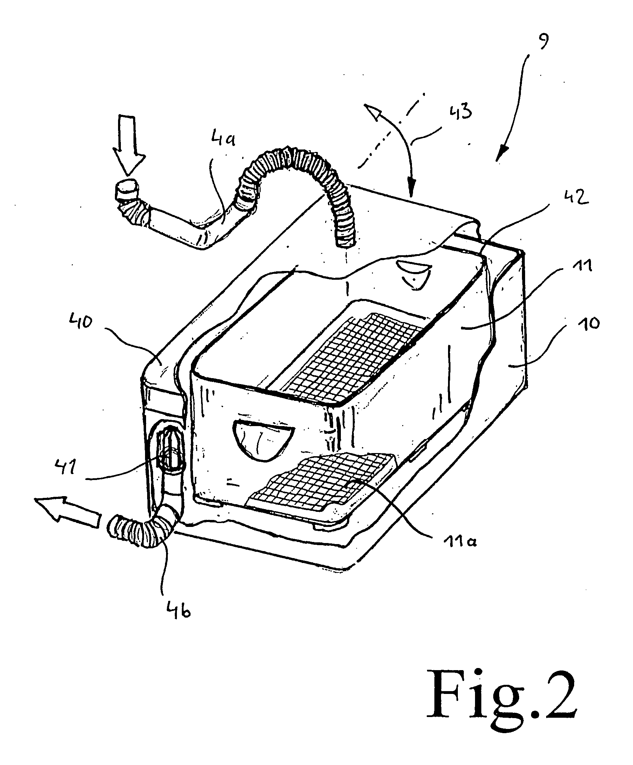

In Fig. 2 ist eine Ausführungsform einer Anreicherungseinrichtung 9 dargestellt, die hier ein, mit einem Deckel 40 verschlossenes Aufnahmegehäuse 10 aufweist. In das Aufnahmegehäuse 10 ist ein Kompostbehälter 11 eingesetzt der einen wasserdurchlässigen Bodenabschnitt 11a aufweist. Das Kompostbehältnis 11 ist weiterhin derart ausgebildet, dass das aus dem Bodenabschnitt 11a abfließende angereicherte Wasser in einen zwischen dem Aufnahmegehäuse 10 und dem Kompostbehältnis 11 gebildeten Zwischenraum hinein austreten und von hier aus über den Ablaufleitungsabschnitt 4b abgesaugt werden kann.

Bei dem hier gezeigten Ausführungsbeispiel ist der Ablaufleitungsabschnitt 4b mit einer Ventileinrichtung 41 versehen durch welche der Ablaufleitungsabschnitt 4b verschlossen wird solange im Inneren des Aufnahmegehäuses 10 ein vorgegebener Füllstand unterschritten wird. Das Kompostbehältnis 11 weist einen oberen Umfangsrand 42 auf, der in abdichtender Weise mit dem Deckel 40 in Eingriff gelangt. Hierdurch wird im Inneren des Kompostbehältnisses 11 im Zusammenspiel mit dem Deckel 40 ein von dem Zwischenraum zwischen Kompostbehältnis 11 und dem verbleibenden Innenraum des Aufnahmegehäuses 10 getrennter Bewässerungsabschnitt gebildet. In diesem Bewässerungsabschnitt kann unter Zwischenschaltung der in Figur 1angedeuteten Siebplatte 13 über den Ableitungsabschnitt 4a Fluid unmittelbar auf die in dem Kompostbehältnis 11 aufgenommene Kompostfüllung (hier nicht dargestellt) geleitet werden.

Die Deckeleinrichtung 40 ist zur Entnahme des Kompostbehältnisses bzw. zum Einsatz mit einer frischen Kompostfüllung beschickten Kompostbehältnisses in eine Offenstellung bringbar wie hier durch das Pfeilsymbol 43 angedeutet.

In Fig. 3 ist ein Abschnitt der im unteren Bereich der Behältereinrichtung 1 vorgesehenen Bodengruppe 2 dargestellt. An die untere Umfangswandung 1a der Behältereinrichtung 1 ist über Schnellverschlusseinrichtung 44 eine Bodenverschlussanordnung 3 angesetzt die mit einer Gaseinleitungseinrichtung 34 versehen ist. Die Gaseinleitungseinrichtung 34 umfasst eine aus einem Porösmaterial hier Synterpolyamid gefertigte Sprudelplatte 45 welche im Bereich ihrer Unterseite mit Druckluft beaufschlagt wird. die Druckluft wird hierbei über eine flache Umfangsnut 46 und die darin angeschlossene, bereits in Verbindung mit Fig. 1 genannte Druckluftleitung 35 zugeführt. Aufgrund des in der Druckluftleitung 35 herrschenden Überdruckes gelangt die zugeführte Druckluft in Form feiner Bläschen 47 in die Flüssigkeit wobei diese Bläschen aufgrund des in der Flüssigkeit herrschenden Auftriebs allmählich nach oben wandern. An die Bodenverschlussanordnung 3 ist die Ablaufleitung 4 angeschlossen über welche wie durch das Pfeilsymbol angedeutet Fluid aus der Behältereinrichtung 1 abgeleitet werden kann. Die Steuerung der Druckluftzufuhr erfolgt vorzugsweise unter Berücksichtigung des momentanen Zustands des in dem Behälter 1 befindlichen Fluides insbesondere in Abhängigkeit von dessen Temperatur, Sauerstoff- und Zuckergehalt.

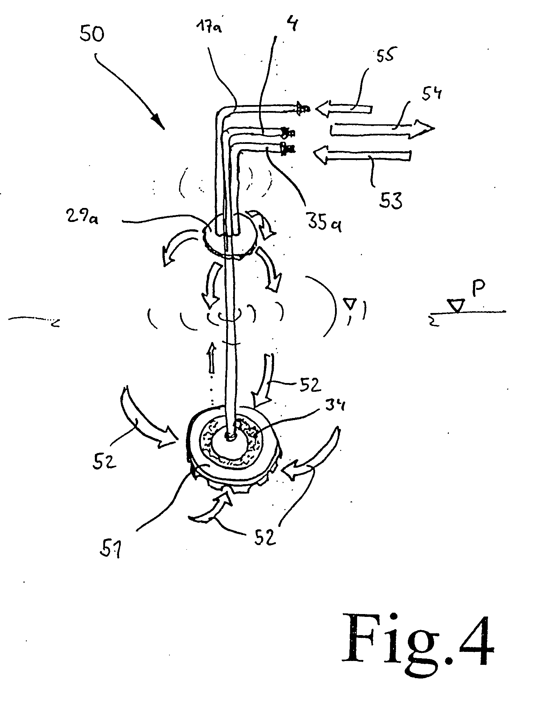

In Fig. 4 ist eine Tauchlanze dargestellt die in eine mit Wasser befüllte Behältereinrichtung einstellbar ist, wobei die zur erfindungsgemäßen Generierung des mit Mikroorganismen befrachteten Wassers erforderlichen Fluidströme über dieses Tauchlanzenelement bereitgestellt werden können. Das Tauchlanzenelement 50 umfasst einen Fussabschnitt 51 über welchen das in dem Behälter befindliche Fluid wie durch die Pfeilsymbole 52 angedeutet angesaugt und über eine Ableitungseinrichtung 4 zu einer Anreicherungseinrichtung geführt werden kann. Im Bereich des Fussabschnitts 51 ist eine Gaseinleitungseinrichtung 34 vorgesehen, wobei die Gaseinleitungseinrichtung 34 über eine Druckluftleitung 35a mit Druckluft versorgt wird. Das Tauchlanzenelement 50 umfasst weiterhin einen Sprühkopf 29a über welchen das über eine Rücklaufleitung 17a zugeführte Fluid in fein verteilter Form oberhalb des Flüssigkeitsspiegels P in fein verteilter Form auf diesen abgesprüht werden kann.

Das Pfeilsymbol 53 veranschaulicht die Gaszufuhr in die Druckluftleitung 35a, das Pfeilsymbol 54 veranschaulicht die Ablichtung des Fluides über den Fussabschnitt aus der Ablaufleitung 4, das Pfeilsymbol 55 beschreibt die Rückleitung des über die Umwälzpumpe 16 (Fig.1) geförderten Fluides nach dessen Durchlauf durch die Anreicherungseinrichtung (z.B nach Fig.2).

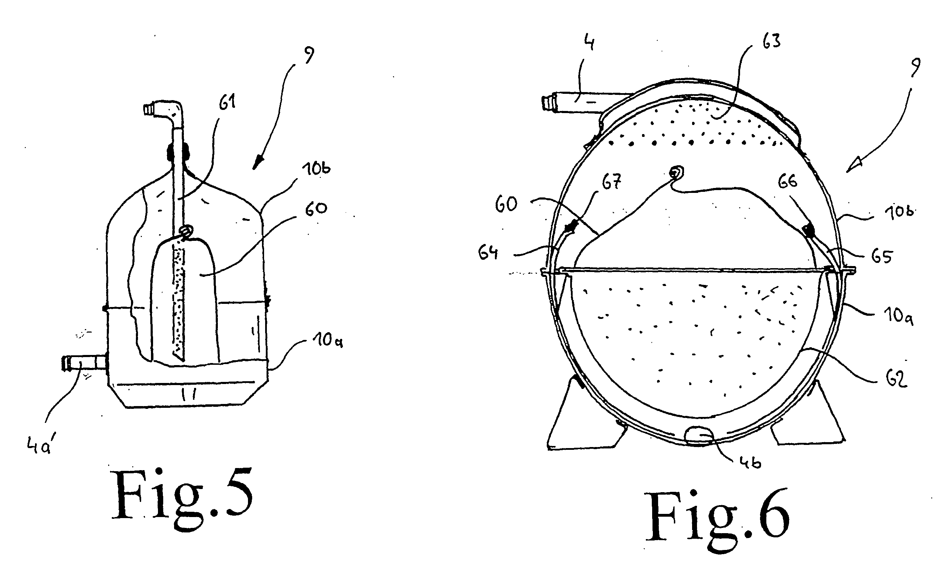

In Fig. 5 ist eine weitere Variante einer Anreicherungseinrichtung 9 dargestellt. Die Anreicherungseinrichtung umfasst ein Aufnahmegehäuse 10 a das hier topfartig ausgebildet ist und mit einem Ablaufleitungsanschluss 4a'versehen ist. In das Aufnahmegehäuse 10a ist ein Kompostbeutel 60 eingesetzt, dessen Wandung aus einem wasserdurchlässigen Material gefertigt ist. In den Kompostbeutel 60 ist eine Spüllanze 61 eingesteckt, über welche der Kompostbeutel 60 von innen heraus mit Wasser durchspült werden kann. Die Spüllanze 61 ist hierzu im Bereich ihrer Aussenumfangsfläche mit einer Vielzahl von Austrittsöffnungen versehen. Die Spüllanze 61 ist in abdichtender Weise durch eine oberen Deckelabschnitt 10b des Aufnahmegehäuses hindurchgeführt. Für den Fall dass gewährleistet ist, dass in dem unteren Bereich des Aufnahmegehäuses 10 ein vorbestimmter Füllstand nicht überschritten wird, kann auf das Deckelement 10b verzichtet werden.

In Fig. 6 ist eine weitere Variante eine Anreicherungseinrichtung dargestellt, die hier ein im wesentlichen kugel- kalottenförmiges Außengehäuse 10 aufweist auf welches ein ebenfalls kalottenförmig ausgebildetes Deckelelement 10b aufgesetzt ist. Im Innenbereich des Aussengehäuses 10a ist ein Kompostkorb 62 angeordnet, in welchen ein aus einem wasserdurchlässigen Gewebe gefertigter, mit Kompost gefüllter Kompostbeutel eingesetzt ist. Der Kompostbeutel 60 wird von einer oberhalb desselben angeordneten Sprühdüsenanordnung 63 mit Wasser beaufschlagt. die Sprühdüsenanordnung wird hier über Ablaufleitung 4 bewässert. Das sich in dem Aufnahmegehäuse 10a nach Durchtritt durch den Kompostbeutel 60 sammelnde Wasser kann über eine, zu einem Ansaugleitungsabschnitt 4b führende Ableitungsöffnung abgeleitet werden.

In vorteilhafter Weise ist es möglich, die in der Anreicherungseinrichtung 9 aufgenommene Kompostfüllung derart mit Sauerstoff insbesondere mit Luft zu versorgen, dass im Inneren der Kompostfüllung überwiegend aerobe Mikroorganismen gebildet werden. Der in diesem Zusammenhang erforderliche Sauerstoff kann über das Bewässerungsfluid (Ablaufleitung 4a) zugeführt werden. In vorteilhafter Weise wird jedoch zusätzlich Druckluft oder gegebenenfalls reiner Sauerstoff in den Kompost eingebracht. Hierzu ist es möglich, insbesondere den oberhalb des Komposts liegenden Bereich mit Drucklluft zu beaufschlagen.

Um weiterhin auch eine weitere Unterstützung der Sauerstoffanreicherung des über den Ablauf 4b abgezogenen Abtropfextraktes zu erreichen sind bei dem Ausführungsbeispiel nach Fig. 6 zusätzlich Luftbypassleitungen 64, 65 vorgesehen, über welche infolge des durch die Umwälzpumpe aufgebauten Unterdruckes Luft in das Abtopfextrakt eingesaugt und in der Umwälzpumpe verwirbelt werden kann. Der durch die Bypassleitungen zugeleitete Luftstrom kann durch Drosseln 66, 67 dosiert werden.

In weiterhin vorteilhafter Weise ist es möglich, die in das Fluid einzubringende Nährlösung insbesondere Melasse im Bereich der Anreicherungseinrichtung oder im Bereich des ausserhalb der Behältereinrichtung 1 liegenden Leitungssystems einzuspeisen.

Unter dem Begriff Anreicherungseinrichtung ist eine Einrichtung zu verstehen durch welche eine Befrachtung des in der Behältereinrichtung 1 befindlichen, überwiegend aus Wasser bestehenden Fluides mit organischen Stoffen, Mikroben, Sporen, Bazillen, Einzellern oder sonstigen Lebewesen erfolgt indem das Wasser mit einem Träger- Brut oder Kompostmedium in Kontakt gebracht wird. Die Vermehrung der Mikroorganismen kann in der Anreicherungseinrichtung und/oder in der Behältereinrichtung 1 erfolgen.

### Die vorangehende Erfindung kann wie nachfolgend erläutert, angewendet werden:

Zur Bereitung eines mit einem gewünschten Spektrum an Mikroorganismen versetzten Fluides, wird zunächst die Behältereinrichtung 1 mit Wasser gefüllt. Über ein Messeinrichtung wird die Temperatur des Wassers erfasst. Durch die Steuereinrichtung 19 wird das Mischventil in eine Stellung gebracht in welches dieses den Leitungsabschnitt 4a vollständig sperrt und eine Fluidströmung aus der Ablaufleitung 4 in den zur Umwälzpumpe 16 führenden Saugleitungsabschnitt 4d freigibt. über die Umwälzpumpe wird das Fluid durch eine in der Rückleitungseinrichtung 17 vorgesehene Heizeinrichtung 23 erwärmt und in erwärmten Zustand über den Sprühkopf 29 auf den Flüssigkeitsspiegel des in der Behältereinrichtung 1 befindlichen Fluides abgesprüht.

Zwischenzeitlich wird die Anreicherungseinrichtung 9 mit Kompost beschickt. Hierzu wird die Deckeleinrichtung der Anreicherungseinrichtung 9 geöffnet und eine, in einem wasserdurchlässigen Sack abgepackte Kompostfüllung 12 in die Anreicherungseinrichtung 9 eingelegt.

Die Anreicherungseinrichtung wird nunmehr geschlossen. Beim Schließen der Anreicherungseinrichtung entsteht oberhalb der Kompostfüllung 12 ein Raumabschnitt der über den Leitungsabschnitt 4a mit Fluid beaufschlagbar ist. Bei Erreichen einer vorgegebenen Grenztemperatur des in der Behältereinrichtung 1 befindlichen Fluides veranlasst die Steuereinrichtung 19 eine Umschaltung des Mischventils 6 in einen Zustand in welchem ein Teilstrom des über die Ablaufleitung 4 aus der Behältereinrichtung 1 abgeleiteten Fluides der Kompostfüllung 12 zugeführt wird.

Unter dem Begriff "Extraktor" ist im vorliegenden Fall auch eine Einrichtung zu verstehen durch welche eine diese durchlaufendes Fluid mit Stoffen, Lebewesen, Mikroben, Sporen, Keimen, Zellen, und/oder Zellkulturen befrachtet wird.

Im Falle einer Befrachtung der Anreicherungseinrichtung mit einer Kompostfüllung ist es möglich, durch die Kompostfüllung das potentielle Spektrum der in dem Fluid enthaltenen Befrachtungsstoffe zu bestimmen. Hierbei handelt es sich vorzugsweise um Bodenbakterien, Actinomyceten, Pilze, Bodenalgen, organische Substanzen, antagonistischen Organismen (Edaphon), Humus, niedere Würmer, Ringelwürmer, Gliederfüßler, Protozoen, Rhizopoden, Flagellaten, Ciliaten.

Das die Kompostfüllung 12 durchfliessende Fluid wird unterhalb der Kompostfüllung 12 aufgefangen, und über den Saugleitungsabschnitt 4d der Umwälzpumpe 16 zugeführt. Das im Zusammenspiel mit der Anreicherungseinrichtung 9, mit Mikroorganismen und gegebenenfalls anderweitigen Bestandteilen befrachtete Fluid gelangt über den Sprühkopf 29 oder auch die Kesselspeisedüse 25 in die Behältereinrichtung 1 zurück. Während das in der Behältereinrichtung 1 befindliche Fluid über die Ablaufleitung 4 und die Rücklaufleitung 17 unter Einschluss der Umwälzpumpe 16 umgewälzt wird, wird das in der Behältereinrichtung befindliche Fluid mit Sauerstoff versetzt. Hierzu wird über die Kompressoreinrichtung 36 Umgebungsluft in die Druckluftleitung 35 gepresst und über eine Polyamid-Sinterplatte in fein verteilter Form im Bodenbereich der Behältereinrichtung 1 in das darin befindliche Fluid eingespeist. Über eine Abzweigung wird in einer, nach Massgabe der Steuereinrichtung 19 gesteuerten Weise auch Druckluft der Anreicherungseinrichtung 9 zugeführt. Hierdurch wird erreicht, dass auch innerhalb der Kompostfüllung 12 Sauerstoff in ausreichendem Masse zur Verfügung steht.

Über die Messanordnung 18 wird permanent der Zustand des durch die Rückleitungseinrichtung 17 strömenden Fluides erfasst. Nach Massgabe eines vorgegebenen Brau-Planes sowie in Abhängigkeit von dem durch die Messanordnung 18 generierten Messsignalen wird über einen Einspeisestutzen 70 eine Nährlösung beispielsweise Melasse in den Fluidkreislauf eingespeist. Die Einspeisung wird vorzugsweise derart vorgenommen, dass ein in Abhängigkeit vom Sauerstoffsättigungsgrad des Fluides abhängiger Wert der Nährlösungskonzentration nicht überschritten wird. Über die Steuereinrichtung 19 wird der Reifegrad der in der Behältereinrichtung 1 befindlichen Lösung erfasst. Sobald seitens der Steuereinrichtung 19 erkannt wird, dass die in der Behältereinrichtung befindliche Lösung einen vorgegebenen Reifegrad erreicht hat, wird ein Signal generiert das über eine Datenleitung einer zentralen Koordinationseinrichtung zugeführt wird.

In Abhängigkeit von der verwendeten Kompostfüllung 12 sowie in Abhängigkeit von der verwendeten Nährlösung sowie auch in Abhängigkeit von dem gewünschten Spektrum an Mikroorganismen in dem bereiteten Fluid können unterschiedliche Prozeduren bei der Generierung des Fluides abgearbeitet werden. Es ist möglich, entsprechende Prozeduren über ein Interface oder durch Herstellung eines Datenverbundes, insbesondere über das Internet über die Steuereinrichtung 19 zu speichern.

Die für den Zustand des bereiteten Fluides indikativen Messwerte können an der Anzeigeeinrichtung 37 visualisiert werden. Auch der zeitliche Ablauf des Fluid-Bereitungsverfahrens kann an der Anzeigeeinrichtung visualisiert werden.

Die Anzeigeeinrichtung 37 ist vorzugsweise als Touch-Pad ausgebildet und ermöglicht gleichzeitig eine Benutzergeführte Eingabe der gewünschten Verfahrensparameter.

Nach Fertigstellung des Fluides, kann dieses über die Entnahmeleitung 21 abgeleitet werden. Während der Entnahme des Fluides kann die Anreicherungseinrichtung 9 mit einer neuen Kompostfüllung 12 beschickt werden. Für den Fall, dass die erfindungsgemäße Vorrichtung nur intervallweise benutzt werden soll, kann die verbrauchte Kompostfüllung 12 entfernt, die Anreicherungseinrichtung ohne erneutes Einsetzen einer Kompostfüllung geschlossen und die gesamte Anlage im Rahmen eines Spülprogrammes gespült werden. Das hierbei generierte Spülwasser kann zu Bewässerungszwecken verwendet werden. Eine besonders wasserverbrauchsarme Spülung der erfindungsgemäßen Vorrichtung, kann erreicht werden, indem anstelle der Kompostfüllung in die Anreicherungseinrichtung eine Filtermaterialfüllung eingesetzt wird. Über diese Filtermaterialfüllung ist es möglich, das in der Behältereinrichtung 1 befindliche Wasser, derart vorzubehandeln, dass dieses sich in besonderem Masse zur Generierung der erfindungsgemäß gewünschten Mikroorganismen eignet. Die Filtermaterialfüllung kann aus kugelförmig oder Pelletartigen Filterkörpern bestehen, die als loses oder in einem durchlässigen Beutel abgepackte Schüttung in die Anreicherungseinrichtung eingebracht werden können.

## Patentansprüche

1. Vorrichtung zur Bereitung eines, mit Mikroorganismen befrachteten Fluides mit:
- einer Behältereinrichtung (1) zur Aufnahme des mit Mikroorganismen befrachteten Fluides,
- einer Ableitungseinrichtung (4) zur Ableitung eines Fluidstromes aus der Behältereinrichtung (1),
- einer Rückleitungseinrichtung (17) zur Rückleitung des über die Ableitungseinrichtung (4) geführten Fluidstromes in die Behältereinrichtung (1), und
- einer Gasbefrachtungseinrichtung zur Befrachtung des in der Behältereinrichtung (1) aufgenommenen Fluides mit Gas, insbesondere Sauerstoff,
- wobei eine Anreicherungseinrichtung (9) vorgesehen ist, *zur Auswaschung von Mikroorganismen aus einem Fermentationsmedium,*
- und zumindest ein Teilstrom des Fluidstromes über die Anreicherungseinrichtung (9) geführt ist,
- und die Anreicherungseinrichtung (9) außerhalb der Behältereinrichtung (1) angeordnet ist
- und die Anreicherungseinrichtung (9) eine Aufnahmekammer aufweist zur Aufnahme des Fermentationsmediums,
- wobei die Anreicherungseinrichtung (9) weiterhin eine Deckeleinrichtung (10b) aufweist, zur Ein- oder Ausbringung des Fermentationsmediums in die, bzw. aus der Aufnahmekammer.

2. Vorrichtung nach Anspruch 1, **dadurch gekennzeichnet, dass** die Anreicherungseinrichtung (9) in der Behältereinrichtung (1) oberhalb eines Flüssigkeitsspiegels (P) angeordnet ist.

3. Vorrichtung nach Anspruch 1, **dadurch gekennzeichnet, dass** das Fermentationsmedium aus einem Kompostmaterial gebildet ist.

4. Vorrichtung nach wenigstens einem der Ansprüche 1 bis 3, **dadurch gekennzeichnet, dass** die Anreicherungseinrichtung 9 eine Rückhalteeinrichtung aufweist, zum Zurückhalten ausgeschwemmter Kompostpartikel.

5. Vorrichtung nach wenigstens einem der Ansprüche 1 bis 4, **dadurch gekennzeichnet, dass** die Anreicherungseinrichtung unterhalb der Behältereinrichtung (1) angeordnet ist.

6. Vorrichtung nach wenigstens einem der Ansprüche 1 bis 5, **dadurch gekennzeichnet, dass** eine Förderpumpe (16) vorgesehen ist, zur Förderung des Fluidstromes.

7. Vorrichtung nach Anspruch 6, **dadurch gekennzeichnet, dass** die Förderpumpe (16) stromabwärts der Anreicherungseinrichtung (9) angeordnet ist.

8. Vorrichtung nach wenigstens einem der Ansprüche 1 bis 7, **dadurch gekennzeichnet, dass** das dem Fermentationsmedium zugeführte Fluid aufgrund des durch das Höhenniveau des Flüssigkeitsspiegels (P) aufgebauten statischenDruckes der Anreicherungseinrichtung (9) zugeführt wird.

9. Vorrichtung nach wenigstens einem der Ansprüche 1 bis 8, **dadurch gekennzeichnet, dass** eine Druckausgleichsvorrichtung vorgesehen ist, zur Begrenzung des Gasdruckes in der Behältereinrichtung (1).

10. Vorrichtung nach wenigstens einem der Ansprüche 1 bis 9, **dadurch gekennzeichnet, dass** die Gasbefrachtungseinrichtung eine Sprudeleinrichtung aufweist, zur Einleitung des Gases in das Fluid in fein verteilter Form.

11. Vorrichtung nach wenigstens einem der Ansprüche 1 bis 10, **dadurch gekennzeichnet, dass** das rückgeleitete Fluid als Fluidstrahl in das im Behälter befindliche Fluid eingestrahlt wird.

12. Vorrichtung nach wenigstens einem der Ansprüche 1 bis 12, **dadurch gekennzeichnet, dass** eine Einspeisungseinrichtung (17) vorgesehen ist, zur Einspeisung einer Nährlösung, insbesondere Melasse.

13. Vorrichtung nach Anspruch 12, **dadurch gekennzeichnet, dass** der Melasseeinspeiser ein Melasseaufnahmebehältnis umfasst, und dass der Innenraum des Melasseaufnahmebehältnisses mit einem Druckmedium beaufschlagbar ist.

14. Vorrichtung nach Anspruch 12 oder 13, **dadurch gekennzeichnet, dass** als Druckmedium Luft verwendet wird.

15. Vorrichtung nach Anspruch 13 oder 14, **dadurch gekennzeichnet, dass** als Druckmedium Wasser verwendet wird.

16. Vorrichtung nach wenigstens einem der Ansprüche 1 bis 15, **dadurch gekennzeichnet, dass** die Melasse in ein Transportverhältnis abgefüllt ist, dass an den Melasseeinspeiser ankoppelbar ist.

17. Vorrichtung nach wenigstens einem der Ansprüche 1 bis 16, **dadurch gekennzeichnet, dass** der Kompost als Beschickungseinheit konfektioniert ist.

18. Vorrichtung nach Anspruch 17, **dadurch gekennzeichnet, dass** der Kompost in ein wasserdurchlässiges Behältnis eingefüllt ist.

19. Vorrichtung nach Anspruch 17 oder 18, **dadurch gekennzeichnet, dass** der Kompost in einen Sack oder Beutel abgefüllt ist.

20. Vorrichtung nach wenigstens einem der Ansprüche 17 bis 19, **dadurch gekennzeichnet, dass** der Kompost in einem kastenartigen Behältnis abgepackt ist das derart bemessen ist, dass dieses in die Anreicherungseinrichtung einsetzbar ist.

## Claims

1. Device for the preparation of a fluid loaded with microorganisms with
- a reservoir (1) to hold the fluid loaded with microorganisms
- an outflow facility (4) to allow fluid to flow out of the reservoir (1)
- a fluid return facility (17) to lead the fluid flowing through the outflow facility (4) into the reservoir (1) and
- a gas charging facility to charge the fluid contained in the reservoir (1) with gas, in particular oxygen
- whereby an enrichment facility (9) is provided to wash out microorganisms from a fermentation medium
- and at least one part flow of the fluid is led through the enrichment facility (9)
- and the enrichment facility (9) is arranged outside of the reservoir (1)
- and the enrichment facility (9) has a chamber to hold the fermentation medium
- whereby the enrichment facility (9) also has a cover (10b) for filling or removing the fermentation medium into or from the chamber.

2. Device in accordance with claim 1 and **characterised by** the fact that the enrichment facility (9) is arranged in the reservoir (1) above a fluid level (P).

3. Device in accordance with claim 1 and **characterised by** the fact that the fermentation medium consists of a compost material.

4. Device in accordance with at least one of the claims 1 to 3 and **characterised by** the fact that the enrichment facility 9 has a retention facility to retain any washed-out compost particles.

5. Device in accordance with at least one of the claims 1 to 4 and **characterised by** the fact that the enrichment facility is arranged below the reservoir (1).

6. Device in accordance with at least one of the claims 1 to 5 and **characterised by** the fact that a pump (16) is provided to convey the fluid.

7. Device in accordance with claim 6 and **characterised by** the fact that the pump (16) is arranged downstream of the enrichment facility (9).

8. Device in accordance with at least one of the claims 1 to 7 and **characterised by** the fact that the fluid fed into the fermentation medium is led into the enrichment facility (9) due to static pressure built up by the height of the fluid level (P).

9. Device in accordance with at least one of the claims 1 to 8 and **characterised by** the fact that a pressure compensation device is provided to limit the gas pressure in the reservoir (1).

10. Device in accordance with at least one of the claims 1 to 9 and **characterised by** the fact that the gas loading device has a bubbling device to feed the gas into the fluid in a finely distributed form.

11. Device in accordance with at least one of the claims 1 to 10 and **characterised by** the fact that the returned fluid is sprayed into the fluid in the reservoir in the form of a jet of fluid.

12. Device in accordance with at least one of the claims 1 to 11 and **characterised by** the fact that a feed device (17) is provided to feed in a nutrient solution, in particular molasses.

13. Device in accordance with claim 12 and **characterised by** the fact that the molasses feeder has a molasses tank and that the inside of the molasses tank can be pressurised.

14. Device in accordance with claim 12 or 13 and **characterised by** the fact that air is used as a pressure medium.

15. Device in accordance with claim 13 or 14 and **characterised by** the fact that water is used as a pressure medium.

16. Device in accordance with at least one of the claims 1 to 15 and **characterised by** the fact that the molasses is filled into a transport tank which can be coupled with the molasses feeder.

17. Device in accordance with at least one of the claims 1 to 16 and **characterised by** the fact that the compost is prepared as a loading unit.

18. Device in accordance with claim 17 and **characterised by** the fact that the compost is filled into a water-permeable container.

19. Device in accordance with claim 17 or 18 and **characterised by** the fact that the compost is filled into a sack or bag.

20. Device in accordance with at least one of the claims 17 to 19 and **characterised by** the fact that the compost is packed in a box-type container which is dimensioned in such a way that it can be placed into the enrichment facility.

## Revendications

1. Dispositif pour préparer un fluide chargé en micro-organismes comprenant :
- un dispositif de conteneur (1) pour recevoir le fluide chargé en micro-organismes,
- un dispositif de dérivation (4) pour dériver un flux de fluide à partir du dispositif de conteneur (1),
- un dispositif de conduite de retour (17) pour retourner le flux de fluide transporté par l'intermédiaire du dispositif de dérivation (4) dans le dispositif de conteneur (1), et un dispositif de chargement en gaz pour charger le fluide reçu dans le dispositif de conteneur (1) en gaz, notamment en oxygène,
- un dispositif d'enrichissement (9) étant prévu pour le lavage de micro-organismes à partir d'un milieu de fermentation,
- et au moins un flux partiel du flux de fluide étant transporté par l'intermédiaire du dispositif d'enrichissement (9),
- et le dispositif d'enrichissement (9) étant disposé en dehors du dispositif de conteneur (1)
- et le dispositif d'enrichissement (9) présentant une chambre de réception pour recevoir le milieu de fermentation,
- le dispositif d'enrichissement (9) présentant par ailleurs un dispositif de couvercle (10b) pour amener ou évacuer le milieu de fermentation dans la ou à partir de la chambre de réception.

2. Dispositif selon la revendication 1, **caractérisé en ce que** le dispositif d'enrichissement (9) dans le dispositif de conteneur (1) est disposé au-dessus d'un niveau de liquide (P).

3. Dispositif selon la revendication 1, **caractérisé en ce que** le milieu de fermentation est formé par une matière de compost.

4. Dispositif selon au moins l'une des revendications 1 à 3, **caractérisé en ce que** le dispositif d'enrichissement (9) dispose d'un dispositif de retenue pour retenir des particules de compost séparés par lavage.

5. Dispositif selon au moins l'une des revendications 1 à 4, **caractérisé en ce que** le dispositif d'enrichissement est disposé au-dessous du dispositif de conteneur (1).

6. Dispositif selon au moins l'une des revendications 1 à 5, **caractérisé en ce qu'**une pompe de circulation (16) est prévue pour faire circuler le flux de fluide.

7. Dispositif selon la revendication 6, **caractérisé en ce que** la pompe de circulation (16) est disposée en aval du dispositif d'enrichissement (9).

8. Dispositif selon au moins l'une des revendications 1 à 7, **caractérisé en ce que** le fluide amené au milieu de fermentation est amené au dispositif d'enrichissement (9) en raison de la pression statique développée par la hauteur du niveau du liquide (P).

9. Dispositif selon au moins l'une des revendications 1 à 8, **caractérisé en ce qu'**un dispositif de compensation de la pression est prévu pour limiter la pression du gaz dans le dispositif de conteneur (1).

10. Dispositif selon au moins l'une des revendications 1 à 9, **caractérisé en ce que** le dispositif de chargement en gaz présente un dispositif d'effervescence pour faire entrer le gaz de manière finement distribué dans le fluide.

11. Dispositif selon au moins l'une des revendications 1 à 10, **caractérisé en ce que** le fluide retourné est injecté comme jet de fluide dans le fluide se trouvant dans le conteneur.

12. Dispositif selon au moins l'une des revendications 1 à 12, **caractérisé en ce qu'**un dispositif d'alimentation (17) est prévu, pour l'amenée d'une solution nutritive, notamment de la mélasse.

13. Dispositif selon la revendication 12, **caractérisé en ce que** le dispositif d'alimentation en mélasse comprend un conteneur recevant la mélasse et qu'un fluide de pression peut agir sur l'espace intérieur du conteneur recevant la mélasse.

14. Dispositif selon les revendications 12 ou 13, **caractérisé en ce que** de l'air est utilisé comme fluide de pression.

15. Dispositif selon les revendications 13 ou 14, **caractérisé en ce que** de l'eau est utilisée comme fluide de pression.

16. Dispositif selon au moins l'une des revendications 1 à 15, **caractérisé en ce que** la mélasse est remplie dans un conteneur de transport qui peut être couplé au dispositif d'alimentation en mélasse.

17. Dispositif selon au moins l'une des revendications 1 à 16, **caractérisé en ce que** le compost est confectionné en unité de chargement.

18. Dispositif selon la revendication 17, **caractérisé en ce que** le compost est rempli dans un conteneur perméable à l'eau.

19. Dispositif selon les revendications 17 ou 18, **caractérisé en ce que** le compost est rempli dans un sac ou un sachet.

20. Dispositif selon au moins l'une des revendications 17 à 19, **caractérisé en ce que** le compost est conditionné dans un récipient à la manière d'une caisse de dimensions telles que celui-ci peut être inséré dans le dispositif d'enrichissement.
